# EUROPEAN PATENT APPLICATION

(11) **EP 4 439 073 A1**
(43) Date of publication of application: **02.10.2024**
(21) Application number: 24166593.4
(22) Date of filing: 26.03.2024
(51) Int. Cl.: G01N 35/00, G01N 33/543, G01N 35/10

(54) **SAMPLE ANALYZER**

(30) Priority: 27.03.2023 CN 202310358739; 15.03.2024 CN 202410312983
(71) Applicant: Shenzhen Mindray Bio-Medical Electronics Co., Ltd., Shenzhen, Guangdong 518057 (CN)
(72) Inventor: FU, Bin, Shenzhen, 518057 (CN); XU, Wenbo, Shenzhen, 518057 (CN); ZHANG, Qiang, Shenzhen, 518057 (CN); CHEN, Yihua, Shenzhen, 518057 (CN); WU, Xu, Shenzhen, 518057 (CN)
(74) Representative: KIPA AB

(57) **Abstract**

The present invention is suitable for the field of in-vitro diagnostic devices and discloses a sample analyzer. The sample analyzer includes a sample dispensing device, a reagent dispensing device, a sample detection device, a magnetic separation device, a substrate dispensing device, a cleaning liquid dispensing device and a controller; a reaction vessel is at least configured to provide a reaction place for a sample and a reagent, so that a first reaction solution containing at least a first antigen-antibody binder is generated by an antigen-antibody binding reaction between a target antigen in the sample and an antibody in the reagent or between a target antibody in the sample and an antigen in the reagent; the magnetic separation device is configured to perform a magnetic separation cleaning operation on the first reaction solution; the cleaning liquid dispensing device is provided with a function of heating and cooling a cleaning liquid, so as to dispense the cleaning liquid that reaches a temperature within a target temperature range through heating or cooling to the first reaction solution. The cleaning liquid dispensing device of the present invention enables the magnetic separation device to suit reagents with strict temperature-sensitive requirements better, and enhances capability of the sample analyzer in suiting the reagents.

## Description

### TECHNICAL FIELD

The present invention relates to in-vitro diagnostic devices, and in particular to a sample analyzer.

### BACKGROUND

In the related technologies, according to different temperature control solutions for cleaning liquids, sample analyzers may be substantially classified into two cleaning liquid supply solutions as follows. First solution: the cleaning liquid outputted from a cleaning liquid container is directly delivered to a place where the cleaning liquid is needed, without any temperature control measures. Second solution: the cleaning liquid outputted from the cleaning liquid container is heated to a preset temperature by a heater, and then the cleaning liquid is delivered to a place where the cleaning liquid is needed. The cleaning liquids supplied by these two solutions still have the following shortcomings in a specific application.
(1) In the first solution, since the temperature of the supplied cleaning liquid is uncontrollable, the solution cannot meet cleaning liquid supply requirements of temperature-sensitive items. For example, the temperature of the cleaning liquid (also referred to as a separation liquid) required by different detection items in an immunoassay analyzer during an magnetic separation cleaning operation may be different, that is, appropriate temperature zones and temperature-sensitive levels of different detection items during the magnetic separation cleaning operation are different, and therefore the temperature of the cleaning liquid may directly affect a magnetic separation effect and finally affect accuracy of detection results of temperature-sensitive items.
(2) In the second solution, although the second solution has a temperature control function of the cleaning liquid, the second solution has only a temperature control function of unidirectional heating the cleaning liquid. Accordingly, the second solution may only control the temperature of the cleaning liquid to be higher than ambient temperature. For example, in an immunoassay analyzer according to related technologies, the temperature of the cleaning liquid (also referred to as a separation liquid) required during the magnetic separation cleaning operation is generally controlled at about 40°C. This temperature control solution may have the following problems in a specific application. 1) The high-temperature cleaning liquid is easily affected by ambient temperature to fluctuate to a certain extent, thereby affecting stability of optical measurement results, further affecting sensitivity of temperature-sensitive items, and further reducing clinical performance of temperature-sensitive items. 2) When the high-temperature cleaning liquid is used to clean a reaction solution in the magnetic separation cleaning operation, a phenomenon of dissociation of an antigen-antibody binder in the reaction solution easily occurs, thus affecting accuracy of detection results. 3) Only unidirectional temperature control of heating is performed. Therefore, on one hand, the immunoassay analyzer cannot meet requirements of development of low-temperature reagents; on the other hand, it may be very difficult to control the temperature of the cleaning liquid accurately, which may induce occurrence of an adverse phenomenon where the temperature of the cleaning liquid is higher than a target temperature range. 4) Ambient temperature is different in different application scenarios. Therefore, in a specific application, a phenomenon where ambient temperature is higher than an appropriate temperature zone of the cleaning liquid required by some detection items may occur, for example, some detection items require the appropriate temperature zone of the cleaning liquid to be below ambient temperature, while in some application scenarios, ambient temperature itself is higher than the appropriate temperature zone of the cleaning liquid required by the detection items, which cannot allow the immunoassay analyzer to control the temperature of the cleaning liquid to be in the appropriate temperature zone, seriously affecting accuracy of sample detection results.

### SUMMARY

A first object of the present invention is to provide a sample analyzer, which is intended to solve the following technical problems present in related technologies, i.e., the cleaning liquid in a magnetic separation device cannot be controlled to be in an appropriate temperature zone, and the phenomenon of dissociation of the antigen-antibody binder easily occurs in the magnetic separation cleaning operation.

In order to achieve the above object, a technical solution according to the present invention is a sample analyzer, the sample analyzer includes a sample dispensing device, a reagent dispensing device, a sample detection device, a magnetic separation device, a substrate dispensing device, a cleaning liquid dispensing device and a controller;
the sample dispensing device is configured to aspirate a sample from a sample vessel and dispense at least a part of the aspirated sample to a reaction vessel;
the reagent dispensing device is configured to aspirate a reagent from a reagent vessel and dispense at least a part of the aspirated reagent to the reaction vessel, the reaction vessel is at least configured to provide a reaction place for the sample and the reagent, so that a first reaction solution containing at least a first antigen-antibody binder is generated by an antigen-antibody binding reaction between a target antigen in the sample and an antibody in the reagent or between a target antibody in the sample and an antigen in the reagent;
the cleaning liquid dispensing device comprises a heat exchanger, a liquid path device and a power device, the heat exchanger is configured to perform heating and cooling respectively under control of the controller, the liquid path device is configured to provide a flow path for a cleaning liquid, and at least a part of the liquid path device is in contact with the heat exchanger, so as to allow the cleaning liquid in the liquid path device to be heated or cooled by the heat exchanger to reach a temperature within a target temperature range, the power device is connected to the liquid path device, to drive, under control of the controller, the liquid path device to aspirate the cleaning liquid from a cleaning liquid container, and drive, under control of the controller, the liquid path device to dispense the cleaning liquid that reaches a temperature within the target temperature range through heating or cooling by the heat exchanger, to the first reaction solution in the magnetic separation device;
the magnetic separation device is configured to perform a magnetic separation cleaning operation on the first reaction solution through the cleaning liquid provided by the cleaning liquid dispensing device that is within the target temperature range, so as to prepare a second reaction solution;
the substrate dispensing device is configured to dispense a luminescent substrate reagent to the second reaction solution prepared by the magnetic separation cleaning operation, so as to prepare a third reaction solution;
the sample detection device is configured to perform an optical signal detection on the third reaction solution;
the controller is configured to control operations of the sample dispensing device, the reagent dispensing device, the sample detection device, the magnetic separation device, the substrate dispensing device and the cleaning liquid dispensing device, and configured to analyze information of an optical signal detected by the sample detection device, so as to obtain a content of the target antigen in the sample or a content of the target antibody in the sample.

As an implementation, the reagent dispensing device may be further configured to dispense a magnetic bead reagent and a marker reagent to the reaction vessel before the magnetic separation cleaning operation, the magnetic bead reagent is provided with the antibody or the antigen, the marker reagent is an antibody reagent provided with a luminescent marker or an antigen reagent provided with a luminescent marker; the first antigen-antibody binder contained in the first reaction solution is generated by an antigen-antibody binding reaction among the sample, the magnetic bead reagent and the marker reagent, and the optical signal detected by the sample detection device is an optical signal generated by the luminescent substrate reagent in the first antigen-antibody binder under catalysis of the luminescent marker; or
the reagent dispensing device may further be configured to dispense a magnetic bead reagent and a marker reagent to the reaction vessel before the magnetic separation cleaning operation, the magnetic bead reagent is provided with the antibody or the antigen, the marker reagent is an antibody reagent provided with a luminescent marker or an antigen reagent provided with a luminescent marker; the first antigen-antibody binder contained in the first reaction solution is generated by an antigen-antibody binding reaction between the sample and the magnetic bead reagent, the first reaction solution further contains a second antigen-antibody binder generated by an antigen-antibody binding reaction between the marker reagent and the magnetic bead reagent, and the optical signal detected by the sample detection device is an optical signal generated by the luminescent substrate reagent in the second antigen-antibody binder under catalysis of the luminescent marker..

As an implementation, the controller may be further configured to control a target temperature range for a magnetic separation cleaning operation for at least one sample detection item to be within 10°C to 35°C.

As an implementation, the controller may be further configured to control the target temperature range for the magnetic separation cleaning operation for the at least one sample detection item to be within 15°C to 30°C.

As an implementation, the heat exchanger may include a housing and a heat exchange component arranged in the housing, the liquid path device may include a heat exchange tube section, and the liquid path device may be at least partially arranged in the housing and in contact with the heat exchange component.

As an implementation, the heat exchanger may include a heat exchange component and a single energy generation component, the single energy generation component may be configured to be switched between cooling and heating under control of the controller, the heat exchange component may be heat-conductively connected to the single energy generation component;

the liquid path device may include a heat exchange tube section, the heat exchange tube section may be in contact with the heat exchange component, so as to enable the cleaning liquid in the heat exchange tube section to exchange heat with the heat exchange component.

As an implementation, the single energy generation component may be a semiconductor cooling fin, the semiconductor cooling fin may be configured to be switched between cooling and heating under control of the controller, the semiconductor cooling fin may be heat-conductively connected to the heat exchange component.

As an implementation, a groove may be formed at a side of the heat exchange component, and the heat exchange tube section may be at least partially embedded in the groove and in contact with an inner wall of the groove; and/or
the heat exchange tube section may be spirally wound on the heat exchange component.

As an implementation, the heat exchanger may further include a heat conductive block and a heat insulation component, the heat conductive block may be in contact with the semiconductor cooling fin and the heat exchange component respectively to conduct heat of the semiconductor cooling fin to the heat exchange component, and the heat insulation component may surround an outer circumference of the heat conductive block to prevent heat of the heat conductive block from dissipating to a surrounding environment; and/or
the heat exchanger may further include a housing, the heat exchange tube section may be wound on the heat exchange component, and the housing surrounds respective outer circumference of the heat exchange tube section and the heat exchange component to prevent heat of the heat exchange tube section and the heat exchange component from dissipating to the surrounding environment.

As an implementation, the cleaning liquid dispensing device may further include a temperature detection component, the temperature detection component may be configured to detect the temperature of the heat exchange component or detect the temperature of the cleaning liquid output through the heat exchange tube section;
the controller may further be configured to control operation of the semiconductor cooling fin according to temperature information fed back by the temperature detection component.

As an implementation, controlling the operation of the semiconductor cooling fin according to the temperature information fed back by the temperature detection component may include while performing a magnetic separation cleaning operation for a sample detection item: controlling the semiconductor cooling fin to perform cooling when the temperature fed back by the temperature detection component is higher than an upper limit value of a target temperature range for the magnetic separation cleaning operation for the sample detection item, so that the heat exchange component cools the cleaning liquid flowing through the heat exchange tube section; and controlling the semiconductor cooling fin to perform heating when the temperature fed back by the temperature detection component is lower than a lower limit value of the target temperature range for the magnetic separation cleaning operation for the sample detection item, so that the heat exchange component heats the cleaning liquid flowing through the heat exchange tube section.

As an implementation, the controller may be further configured to control the magnetic separation device to perform a magnetic separation cleaning operation for each of one or more detection items of a first type and then control the magnetic separation device to perform a magnetic separation cleaning operation for each of one or more detection items of a second type, when a plurality of sample detection items for a same batch of samples are to be performed,
the one or more detection items of the first type and the one or more detection items of the second type meet at least one of conditions as follows: a temperature control requirement of the one or more detection items of the first type is lower than that of the one or more detection items of the second type; or a difference between a target temperature range required to perform the magnetic separation cleaning operation for each of the one or more detection items of the first type and ambient temperature is smaller than a difference between a target temperature range required to perform the magnetic separation cleaning operation for each of the one or more detection items of the second type and ambient temperature.

As an implementation, the liquid path device may include a main branch, a backflow branch, a liquid injection branch and a switching device, one end of the main branch may be configured to aspirate the cleaning liquid from the cleaning liquid container, the other end of the main branch is connected to the switching device that switches between the backflow branch and the liquid injection branch, and at least a part of the main branch may forms a heat exchange tube section in contact with the heat exchanger, so as to allow the cleaning liquid in the heat exchange tube section to be heated or cooled by the heat exchanger;
the power device may be connected to the main branch;
the liquid injection branch may be configured to dispense the cleaning liquid;
the backflow branch may be configured to form a circulation loop with the main branch and the cleaning liquid container, or the backflow branch may form a circulation loop with the main branch, so that the cleaning liquid outputted from the main branch is guided by the backflow branch to circulate through the heat exchanger, so as to exchange heat.

As an implementation, a liquid path formed by the liquid injection branch may have a length less than or equal to 200 mm.

As an implementation, the liquid path formed by the liquid injection branch may have a length less than or equal to 20 mm.

As an implementation, a volume of the liquid injection branch may be less than or equal to 10% of a liquid amount of the cleaning liquid that is dispensed by the liquid injection branch into the first reaction solution in the magnetic separation device at one time.

As an implementation, a volume of the liquid injection branch may be less than or equal to 5% of the liquid amount of the cleaning liquid that is dispensed by the liquid injection branch into the first reaction solution in the magnetic separation device at one time.

As an implementation, the switching device may be formed with a liquid inlet, a backflow port and a liquid outlet, the liquid inlet may be configured to enable the cleaning liquid after heat exchange through the heat exchanger to flow into the switching device, the backflow port may be configured to enable the cleaning liquid flowing into the switching device to flow back to the main branch through the backflow branch, so as to exchange heat with the heat exchanger, and the liquid outlet is configured to output the cleaning liquid to the liquid injection branch;
the liquid path device may further includes a confluence component, the switching device may be mounted on the confluence component, the liquid outlet may be abutted against the confluence component, the liquid injection branch may include a liquid injection guide channel formed in the confluence component and a first liquid injection component at least partially arranged outside the confluence component to dispense the cleaning liquid, and each of two ends of the liquid injection guide channel may be communicated with a respective one of the liquid outlet and the first liquid injection component.

As an implementation, the switching device may be a multi-way valve, the backflow port is also abutted against the confluence component, the backflow branch may include a backflow guide channel formed in the confluence component and a backflow pipeline at least partially arranged outside the confluence component, each of two ends of the backflow guide channel may be communicated with a respective one of an input end of the backflow pipeline and the backflow port respectively, so that the cleaning liquid flowing out of the backflow port flows to the backflow pipeline through the backflow guide channel, and an output end of the backflow pipeline is communicated with the cleaning liquid container or the main branch; and/or
the switching device may be a multi-way valve, the liquid inlet may also be abutted against the confluence component, the main branch includes a liquid inlet guide channel formed in the confluence component and a main liquid delivery pipeline arranged outside the confluence component, the main liquid delivery pipeline extends from the cleaning liquid container to the liquid inlet guide channel, at least a part of the main liquid delivery pipeline forms the heat exchange tube section, each of two ends of the liquid inlet guide channel is communicated with a respective one of an output end of the main liquid delivery pipeline and the liquid inlet respectively, so that the cleaning liquid flowing out of the main liquid delivery pipeline flows to the switching device through the liquid inlet guide channel.

As an implementation, an orthographic projection of the liquid outlet on the confluence component may not overlap with an orthographic projection of the first liquid injection component on the confluence component; the liquid injection guide channel may include a first channel part and a second channel part, one end of the first channel part is communicated with the liquid outlet, the second channel part may extends from the other end of the first channel part to the first liquid injection component, and an angle greater than 0° and less than 180°may be formed at a junction of the first channel part and the second channel part; and/or
the first liquid injection component may be arranged obliquely relative to a vertical direction.

As an implementation, the liquid path device may further include a main branch, a confluence component and a liquid injection branch, the liquid injection branch may include a liquid injection guide channel formed in the confluence component and a first liquid injection component at least partially arranged outside the confluence component to dispense the cleaning liquid, the main branch may be communicated with the first liquid injection component through the confluence component;
the first liquid injection component may penetrate into the confluence component to dispense the cleaning liquid that reaches a temperature within the target temperature range through heating or cooling by the heat exchanger and that is delivered to the confluence component, into the first reaction solution located in the magnetic separation device,
the first liquid injection component may be a liquid injection needle or a liquid injection tube.

As an implementation, the magnetic separation device may include a magnetic separation disk and a lid, the magnetic separation disk may be configured to accommodate multiple reaction vessels;
the lid may cover the magnetic separation disk;
the liquid path device may further include a main branch, a confluence component and a liquid injection branch, the liquid injection branch may include a liquid injection guide channel formed in the confluence component and a first liquid injection component at least partially arranged outside the confluence component to dispense the cleaning liquid, the main branch may be communicated with the first liquid injection component through the confluence component;
the confluence component may be mounted on the lid.

As an implementation, the controller may be further configured to configure different temperature control parameters for at least two sample detection items according to different sample detection items and/or different liquid injection amounts of the cleaning liquid;
the different temperature control parameters include at least one of different parameters as follows: operation power of the heat exchanger, a liquid amount of the cleaning liquid flowing back through a backflow branch before dispensing the cleaning liquid to the first reaction solution while performing a single sample detection item, or a liquid amount of the cleaning liquid flowing through the heat exchanger before dispensing the cleaning liquid to the first reaction solution while performing the single sample detection item.

As an implementation, the controller may be further configured to configure different temperature control parameters for at least two sample detection items according to different ambient temperatures;
the different temperature control parameters may includ at least one of different parameters as follows: operation power of the heat exchanger, a liquid amount of the cleaning liquid flowing back through a backflow branch before dispensing the cleaning liquid to the first reaction solution of a first sample detection item while performing multiple sample detection items for a same batch of samples, a liquid amount of the cleaning liquid flowing through the heat exchanger before dispensing the cleaning liquid to the first reaction solution of the first sample detection item while performing multiple sample detection items for the same batch of samples, a liquid amount of the cleaning liquid flowing through the backflow branch while performing multiple sample detection items for the same batch of samples, or a frequency of the cleaning liquid flowing through the backflow branch while performing multiple sample detection items for the same batch of samples.

As an implementation, the magnetic separation device may include a holding assembly, a transfer assembly, at least one liquid aspiration assembly, at least one first magnetic assembly and at least one second magnetic assembly, the first magnetic assembly may include (or, be composed of) a pair of first magnetic devices arranged at an interval, a first magnetic attraction position may be formed in between the pair of first magnetic devices arranged at the interval or at a same side of the pair of first magnetic devices arranged at the interval, and the first magnetic assembly may be configured to perform a first magnetic attraction action on the first reaction solution in a reaction vessel located at the first magnetic attraction position, so as to form two spaced magnetic bead groups on an inner side wall of the reaction vessel located at the first magnetic attraction position by attraction;
the second magnetic assembly may be a single second magnetic device, a first liquid aspiration position may be formed at a side of the single second magnetic device, the second magnetic assembly may be configured to perform a second magnetic attraction action on the first reaction solution in a reaction vessel located at the first liquid aspiration position, to form a single magnetic bead group on an inner side wall of the reaction vessel located at the first liquid aspiration position by attraction;
the liquid aspiration assembly may be configured to perform a first liquid aspiration action on the first reaction solution in the reaction vessel located at the first liquid aspiration position;
the holding assembly may be at least configured to hold the reaction vessel located at the first magnetic attraction position and hold the reaction vessel located at the first liquid aspiration position;
the transfer assembly may be at least configured to transfer a reaction vessel loaded with the first reaction solution to the first magnetic attraction position, and may transfer a reaction vessel after completing the first magnetic attraction action to the first liquid aspiration position.

As an implementation, the two first magnetic devices may be arranged at opposite sides of the first magnetic attraction position at an interval in a horizontal direction; and/or
the first magnetic attraction position may be configured to accommodate the reaction vessel, and may perform the first magnetic attraction action while not performing the liquid aspiration action, and the first liquid aspiration position may be configured to accommodate the reaction vessel, and may perform the second magnetic attraction action and the first liquid aspiration action.

A second object of the present invention is to provide a sample analyzer, the sample analyzer includes a sample dispensing device, a reagent dispensing device, a sample detection device, a cleaning liquid dispensing device and a controller,
the sample dispensing device is configured to aspirate a sample from a sample vessel and dispense at least a part of the aspirated sample to a reaction vessel;
the reagent dispensing device is configured to aspirate a reagent from a reagent vessel and dispense the aspirated reagent to the reaction vessel;
the sample detection device is configured to detect a reaction solution made of at least the sample and the reagent;
the cleaning liquid dispensing device comprises a heat exchanger, a liquid path device and a power device, the heat exchanger is configured to perform heating and cooling respectively under control of the controller, the liquid path device is configured to provide a flow path for a cleaning liquid, and at least a part of the liquid path device is in contact with the heat exchanger, so as to allow the cleaning liquid in the liquid path device to be heated or cooled by the heat exchanger to reach a temperature within a target temperature range, the power device is connected to the liquid path device, to drive, under control of the controller, the liquid path device to aspirate the cleaning liquid from a cleaning liquid container, and drive, under control of the controller, the liquid path device to dispense the cleaning liquid that reaches a temperature within the target temperature range through heating or cooling by the heat exchanger, to the first reaction solution in the magnetic separation device;
the controller is configured to control operations of the sample dispensing device, the reagent dispensing device, the sample detection device and the cleaning liquid dispensing device, and configured to analyze information detected by the sample detection device, so as to obtain a detection result,
wherein the liquid path device comprises a main branch, a backflow branch, a liquid injection branch and a switching device, one end of the main branch is configured to aspirate the cleaning liquid from the cleaning liquid container, the other end of the main branch is connected to the switching device that switches between the backflow branch and the liquid injection branch, and at least a part of the main branch forms a heat exchange tube section in contact with the heat exchanger, so as to allow the cleaning liquid in the heat exchange tube section to be heated or cooled by the heat exchanger;
the power device is connected to the main branch;
the liquid injection branch is configured to dispense the cleaning liquid;
the backflow branch forms a circulation loop with the main branch and the cleaning liquid container, or the backflow branch forms a circulation loop with the main branch, so that the cleaning liquid outputted from the main branch is guided by the backflow branch to circulate through the heat exchanger, so as to exchange heat.

As an implementation, a liquid path formed by the liquid injection branch may have a length less than or equal to 200 mm; and/or
a volume of the liquid injection branch may be less than or equal to 10% of a liquid amount of the cleaning liquid that is dispensed by the liquid injection branch at one time.

As an implementation, the switching device may be formed with a liquid inlet, a backflow port and a liquid outlet, the liquid inlet may be configured to enable the cleaning liquid after heat exchange through the heat exchanger to flow into the switching device, the backflow port may be configured to enable the cleaning liquid flowing into the switching device to flow back to the main branch through the backflow branch, so as to exchange heat with the heat exchanger, and the liquid outlet is configured to output the cleaning liquid to the liquid injection branch;
the liquid path device may further include a confluence component, the switching device may be mounted on the confluence component, the liquid outlet may be abutted against the confluence component, the liquid injection branch may include a liquid injection guide channel formed in the confluence component and a first liquid injection component at least partially arranged outside the confluence component to dispense the cleaning liquid, and each of two ends of the liquid injection guide channel may be communicated with a respective one of the liquid outlet and the first liquid injection component.

As an implementation, the controller may be further configured to control the target temperature range for at least one sample detection item to be within 10°C to 35°C.

A third object of the present invention is to provide a sample analyzer, the sample analyzer includes a sample dispensing device, a reagent dispensing device, a sample detection device, a magnetic separation device, a substrate dispensing device, a cleaning liquid dispensing device and a controller,
the sample dispensing device is configured to aspirate a sample from a sample vessel and dispense at least a part of the aspirated sample to a reaction vessel;
the reagent dispensing device is configured to aspirate a reagent from a reagent vessel and dispense at least a part of the aspirated reagent to the reaction vessel, the reaction vessel is at least configured to provide a reaction place for the sample and the reagent, so that a first reaction solution containing at least a first antigen-antibody binder is generated by an antigen-antibody binding reaction between a target antigen in the sample and an antibody in the reagent or between a target antibody in the sample and an antigen in the reagent;
the cleaning liquid dispensing device is configured to provide a cleaning liquid within a target temperature range;
the magnetic separation device is configured to perform a magnetic separation cleaning operation on the first reaction solution through the cleaning liquid provided by the cleaning liquid dispensing device that is within the target temperature range, so as to prepare a second reaction solution;
the substrate dispensing device is configured to dispense a luminescent substrate reagent to the second reaction solution prepared by the magnetic separation cleaning operation, so as to prepare a third reaction solution;
the sample detection device is configured to perform an optical signal detection on the third reaction solution;
the controller is configured to control operations of the sample dispensing device, the reagent dispensing device, the sample detection device, the magnetic separation device, the substrate dispensing device and the cleaning liquid dispensing device, and configured to analyze information of an optical signal detected by the sample detection device, so as to obtain a content of the target antigen in the sample or a content of the target antibody in the sample;
the controller is further configured to control a target temperature range for a magnetic separation cleaning operation for at least one sample detection item to be within 10°C to 35°C.

As an implementation, the controller may be further configured to control the target temperature range for the magnetic separation cleaning operation for the at least one sample detection item to be within 15°C to 30°C; and/or
the cleaning liquid dispensing device is provided with at least a function of cooling the cleaning liquid circularly to make the cleaning liquid reach a temperature within the target temperature range.

As an implementation, the reagent dispensing device may be further configured to dispense a magnetic bead reagent and a marker reagent to the reaction vessel before the magnetic separation cleaning operation, the magnetic bead reagent may be provided with the antibody or the antigen, the marker reagent is an antibody reagent provided with a luminescent marker or an antigen reagent provided with a luminescent marker; the first antigen-antibody binder contained in the first reaction solution may be generated by an antigen-antibody binding reaction among the sample, the magnetic bead reagent and the marker reagent, and the optical signal detected by the sample detection device may be an optical signal generated by the luminescent substrate reagent in the first antigen-antibody binder under catalysis of the luminescent marker; or
the reagent dispensing device may be further configured to dispense a magnetic bead reagent and a marker reagent to the reaction vessel before the magnetic separation cleaning operation, the magnetic bead reagent may be provided with an antibody or an antigen, the marker reagent may be an antibody reagent provided with a luminescent marker or an antigen reagent provided with a luminescent marker; the first antigen-antibody binder contained in the first reaction solution may be generated by an antigen-antibody binding reaction between the sample and the magnetic bead reagent, the first reaction solution may further contain a second antigen-antibody binder generated by an antigen-antibody binding reaction between the marker reagent and the magnetic bead reagent, and the optical signal detected by the sample detection device may be an optical signal generated by the luminescent substrate reagent in the second antigen-antibody binder under catalysis of the luminescent marker.

According to the sample analyzer provided in the present invention, the magnetic separation cleaning operation is performed by the magnetic separation device on the first reaction solution generated by the antigen-antibody binding reaction between the sample and the reagent, to prepare the second reaction solution, an optical signal detection is performed on the third reaction solution prepared from the second reaction solution by the sample detection device, and information of the optical signal detected by the sample detection device is analyzed by the controller, to obtain the detection result of the sample, thereby achieving a function of detecting an immunoassay item. The cleaning liquid that is needed by the magnetic separation cleaning operation and reaches a temperature within the target temperature range is provided by the cleaning liquid dispensing device to the first reaction solution in the magnetic separation device. The cleaning liquid dispensing device is provided with the function of heating the cleaning liquid and the function of cooling the cleaning liquid, that is, the cleaning liquid dispensing device is provided with a function of bidirectional temperature control of the cleaning liquid. Therefore, on one hand, the cleaning liquid dispensing device may supply the cleaning liquid within different temperature ranges according to different detection items and different reagents, so that the cleaning liquid can be controlled to be in an appropriate temperature zone under different application scenarios, which especially meets requirements of some detection items that the cleaning liquid should be lower than ambient temperature, so that the magnetic separation device can suit reagents with strict temperature-sensitive requirements better, enhancing capability of the sample analyzer in suiting the reagents. On the other hand, compared to unidirectional temperature control, bidirectional temperature control may result in a more accurate control of the temperature of the cleaning liquid, which facilitates avoiding an adverse phenomenon that the temperature of the cleaning liquid after being subjected to temperature control exceeds the target temperature range. Furthermore, the cleaning liquid dispensing device is provided with the function of cooling the cleaning liquid, and therefore, the phenomenon of dissociation of the antigen-antibody binder can be reduced by lowering the temperature of the cleaning liquid.

### BRIEF DESCRIPTION OF THE DRAWINGS

In order to explain technical solutions of embodiments of the present invention or the related art more clearly, drawings to be used in descriptions of the embodiments or the related art will be briefly described below. It is apparent that the drawings described below are only some embodiments of the present invention, and other drawings may also be obtained by those of ordinary skill in the art according to structures shown in these drawings without any creative work.
FIG. 1 is a schematic diagram of composition of a sample analyzer according to a first embodiment of the present invention.
FIG. 2 is a schematic diagram of internal distribution of the sample analyzer according to the first embodiment of the present invention.
FIG. 3 is a schematic diagram of a liquid path of a cleaning liquid dispensing device according to the first embodiment of the present invention.
FIG. 4 is a schematic diagram of assembly of a cleaning liquid dispensing device and a magnetic separation device according to the first embodiment of the present invention.
FIG. 5 is a schematic diagram of three-dimensional assembly of a confluence component, a switching device, a first liquid injection component and a liquid aspiration component according to the first embodiment of the present invention, from a visual angle.
FIG. 6 is a schematic diagram of a front-viewed cross section of FIG. 5.
FIG. 7 is a schematic diagram of three-dimensional assembly of a confluence component, a switching device, a first liquid injection component and a liquid aspiration component according to the first embodiment of the present invention, in another view.
FIG. 8 is a schematic exploded view of a heat exchanger and a temperature detection component according to the first embodiment of the present invention.
FIG. 9 is a schematic diagram of an assembly cross section of a heat exchanger according to the first embodiment of the present invention.
FIG. 10 is a schematic diagram of distribution of magnets on a magnetic separation device according to the first embodiment of the present invention.
FIG. 11 is a schematic diagram of a first magnetic attraction position, distribution of magnetic components at the first liquid aspiration position, and distribution of magnetic bead groups formed by attraction according to the first embodiment of the present invention.

### Descriptions of reference numbers

10: sample analyzer; 100: sample dispensing device; 200: reagent dispensing device; 300: sample detection device; 400: cleaning liquid dispensing device; 410: heat exchanger; 411: energy generation component; 4111: semiconductor cooling fin; 412: heat exchange component; 4121: groove; 413: heat conductive block; 414: heat insulation component; 415: housing; 416: heat sink; 417: heat dissipation fan; 420: liquid path device; 421: confluence component; 422: main branch; 4221: main liquid delivery pipeline; 4222: liquid inlet guide channel; 4201: heat exchange tube section; 4202: first tube; 4203: second tube; 4204: third tube; 4205: fourth tube; 4206: fifth tube; 423: liquid injection branch; 4231: first liquid injection component; 4232: liquid injection guide channel; 4207: first channel part; 4208: second channel part; 424: switching device; 425: backflow branch; 4251: backflow pipeline; 4252: backflow guide channel; 426: liquid inlet valve; 427: liquid injection valve; 430: power device; 440: temperature detection component; 500: magnetic separation device; 510: magnetic separation disk; 520: lid; 530: liquid aspiration assembly; 531: liquid aspiration component; 540: first magnetic assembly; 541: first magnetic device; 5411: first magnet; 550: second magnetic assembly; 551: second magnetic device; 5511: second magnet; 560: third magnetic assembly; 561: third magnetic device; 501: first magnetic attraction position; 502: first liquid aspiration position; 503: second magnetic attraction position; 600: sample management device; 700: sample delivery device; 800: reagent storage device; 900: incubation device; 101: substrate dispensing device; 102: controller; 103: reaction vessel supply device; 104: transfer device; 105: reaction vessel recovery device; 20: reaction vessel; 30: cleaning liquid container; 40: magnetic bead group; a: liquid inlet; b: liquid outlet; c: backflow port.

### DETAILED DESCRIPTION

The technical solutions of the embodiments of the present invention will be clearly and fully described below with reference to the drawings in the embodiments of the present invention, and it is apparent that the described embodiments are only part of the embodiments of the present invention, rather than all of the embodiments of the present invention. Based on the embodiments of the present invention, all other embodiments obtained by those of ordinary skill in the art without paying any creative work fall within the scope of protection of the present invention.

The technical solutions of various embodiments may be combined with each other, however, the combination must be based on that those of ordinary skill in the art may implement it. When a combination of the technical solutions is contradictory or cannot be implemented, it should be considered that the combination of the technical solutions does not exist and does not fall within the scope of protection of the present invention either.

The sample analyzer according to the embodiments of the present invention may be an immunoassay analyzer, or may be an All-in-one (AIO) machine with an immunoassay detection function (for example, a biochemical immunoassay AIO machine, a coagulation immunoassay AIO machine, a routine blood immunoassay AIO machine, or an erythrocyte sedimentation rate (ESR) immunoassay AIO machine, etc.), or may be other analyzers requiring temperature control of a cleaning liquid.

### First embodiment:

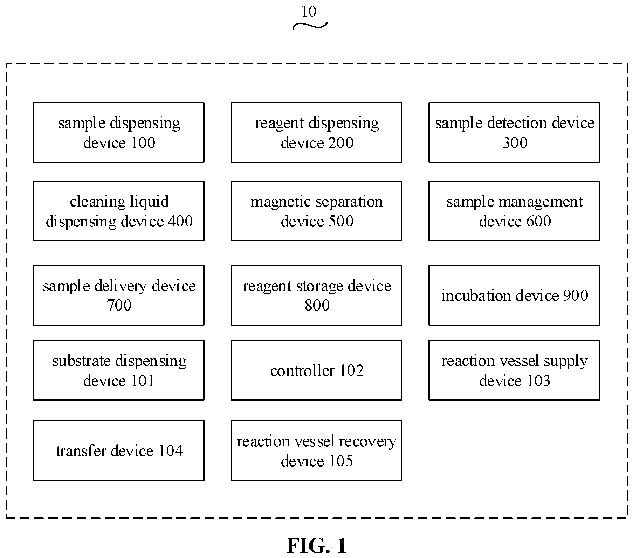

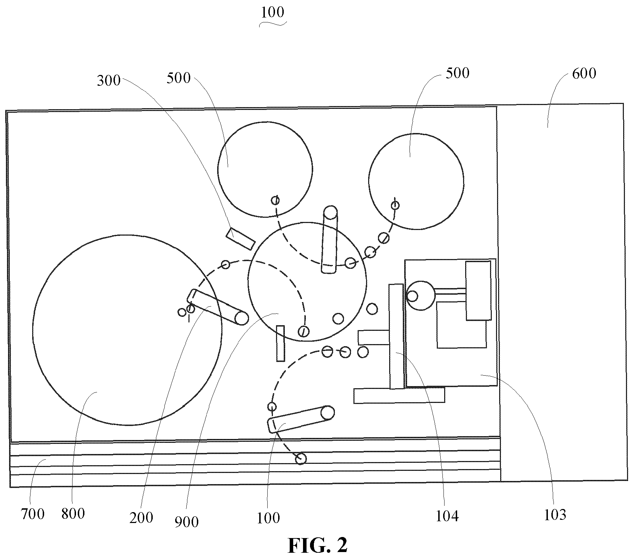

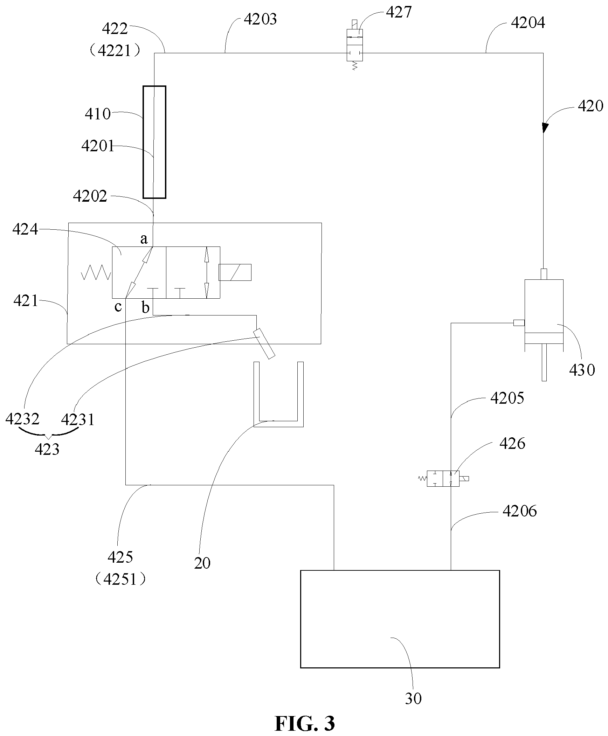

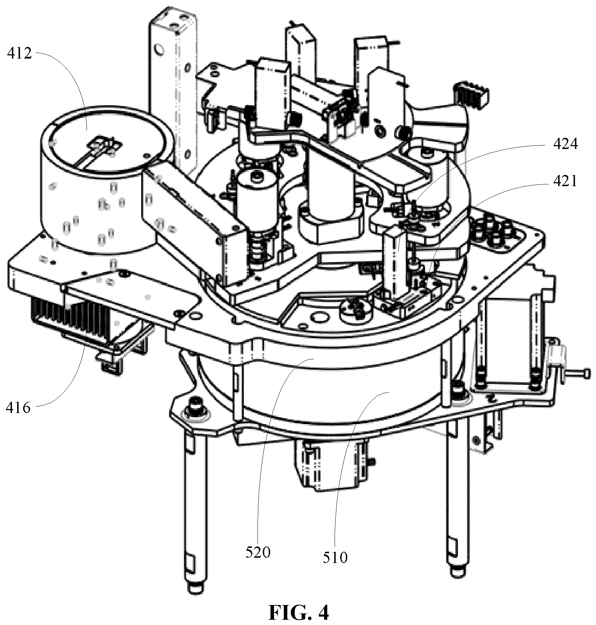

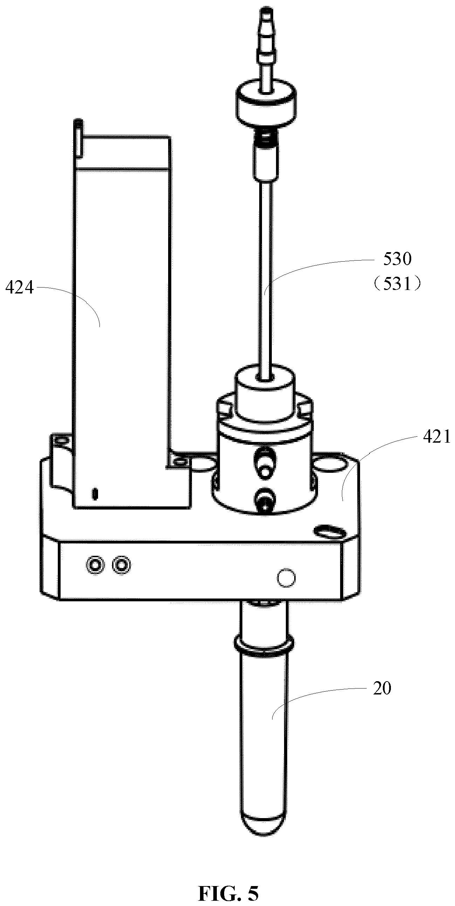

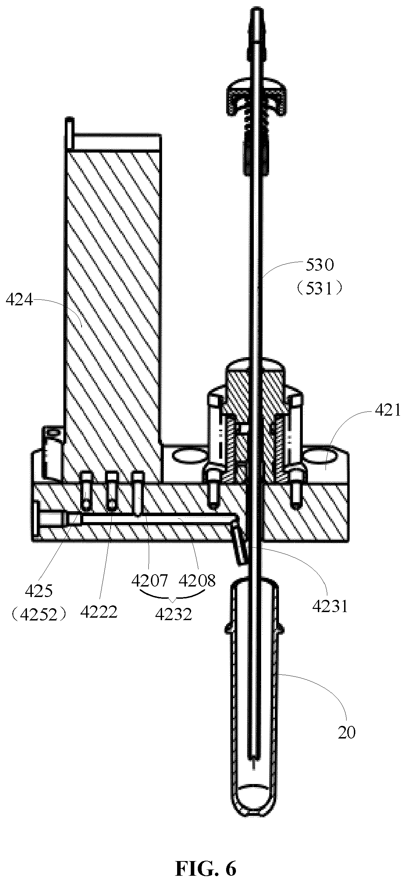

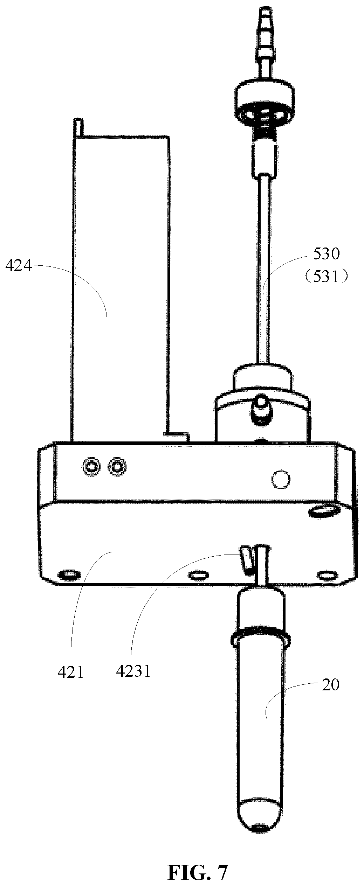

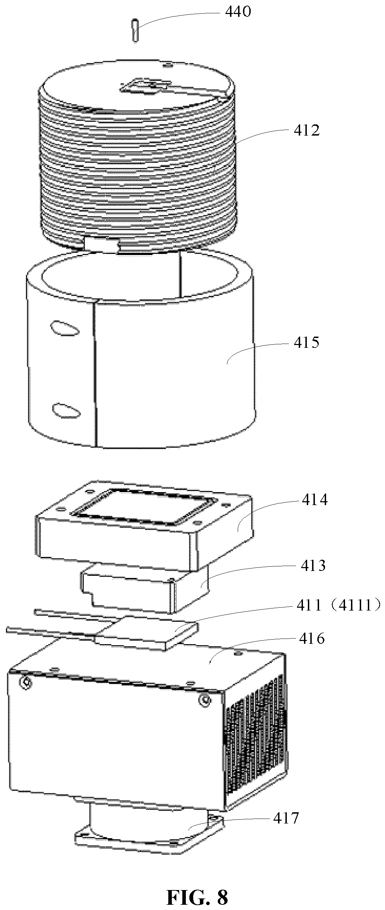

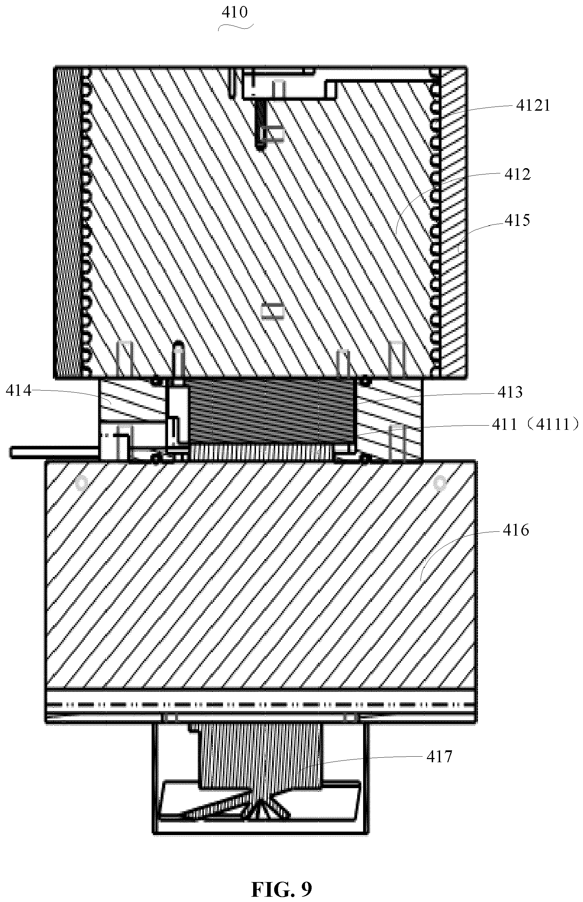

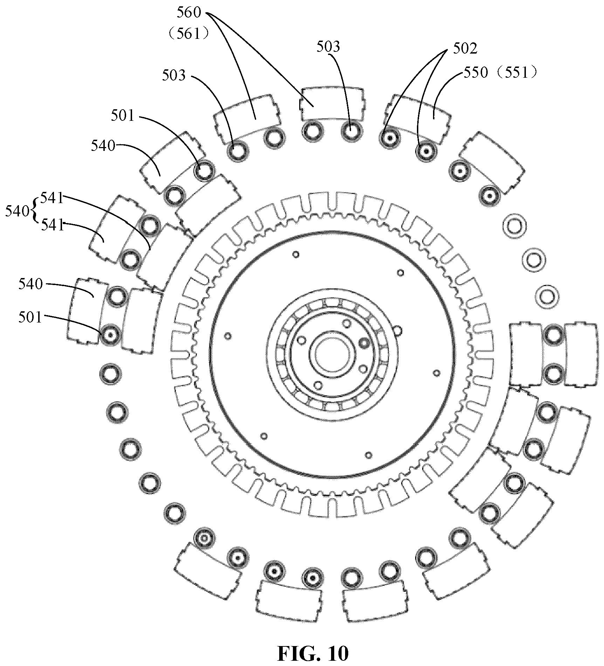

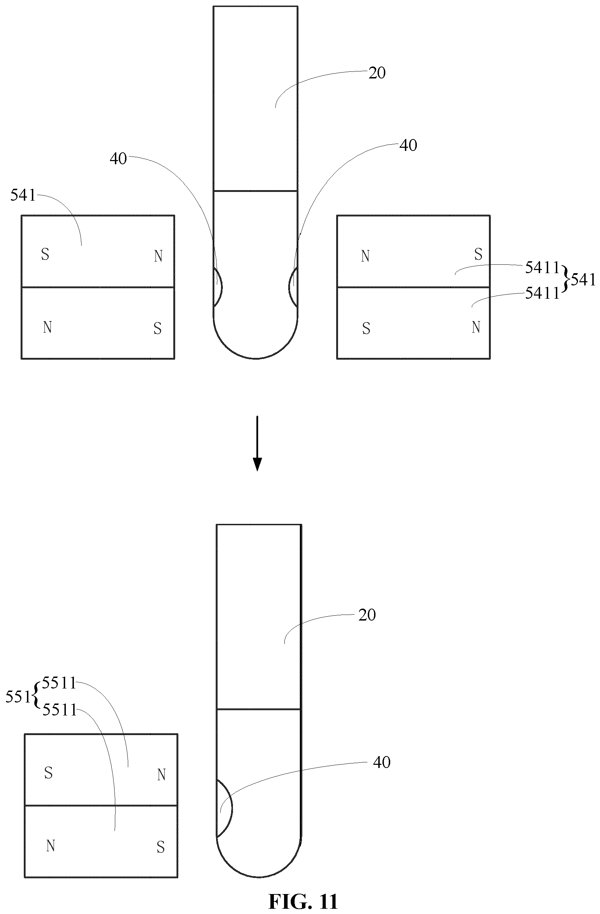

As shown in FIG. 1 to FIG. 11, a sample analyzer 10 according to the first embodiment of the present invention includes a sample dispensing device 100, a reagent dispensing device 200, a sample detection device 300, a cleaning liquid dispensing device 400 and a controller 102. The sample dispensing device 100 is configured to implement a function of dispensing a sample. The reagent dispensing device 200 is configured to implement a function of dispensing a reagent. The sample detection device 300 is configured to detect a reaction solution made of at least the sample and the reagent. The cleaning liquid dispensing device 400 is configured to implement a function of supplying a cleaning liquid. The controller 102 is at least configured to analyze information detected by the sample detection device 300 to obtain a detection result of the sample.

As an implementation, the sample dispensing device 100 is configured to aspirate the sample from a sample vessel and dispense at least a part of the aspirated sample to a reaction vessel 20. The sample vessel is configured to load the sample for sample loading, that is, samples are loaded in the sample analyzer 10 with the sample vessel as a carrier. The reaction vessel 20 is configured to provide a reaction place for the sample. In this implementation, the sample dispensing device 100 is used to aspirate the sample from the sample vessel and dispense the sample to the reaction vessel 20. On one hand, it is beneficial to achieve accurate quantification of the sample, and on the other hand, it is beneficial to dispense the sample in the sample vessel to different reaction vessels 20, to perform different detection items.

As an implementation, the sample vessel is configured to load the sample acquired from a patient, an outer side of the sample vessel is provided with an identification code, and the identification code is associated with at least information of the patient and/or a sample detection item(s). The identification code includes at least one of a bar code, a Quick Response (QR) code or a Radio Frequency Identification (RFID) code.

As an implementation, the sample dispensing device 100 includes a sample needle, a first aspiration and discharge power device and a first movement power device. The first aspiration and discharge power device is connected to the sample needle through a pipeline, to drive the sample needle to perform aspiration and discharge actions, thereby achieving functions of aspirating and discharging the sample by the sample needle. The first movement power device is configured to drive the sample needle to perform two-dimensional or three-dimensional spatial movement, to drive the sample needle to move to different operation positions respectively, such as a sample aspiration position, a sample loading position, a cleaning position, a standby position of the sample needle, etc. The sample dispensing device 100 is configured to aspirate the sample from a sample vessel located at the sample aspiration position and dispense the sample to a reaction vessel 20 located at the sample loading position.

As an implementation, the sample analyzer 10 further includes a sample management device 600 and a sample delivery device 700. The sample management device 600 is configured to manage sample vessels to implement at least loading of the sample. The sample delivery device 700 is configured to deliver the sample vessels outputted from the sample management device 600 to the sample aspiration position. The sample management device 600 may implement a function of batch loading of sample vessels, and specifically, an operator or an operation robot may place sample vessels loaded with samples on the sample management device 600, the sample management device 600 is configured to store sample vessels and transfer the sample vessels loaded with samples to the sample delivery device 700. Of course, in a specific application, the sample analyzer 10 may not be provided with the sample management device 600 and the sample delivery device 700. For example, as an alternative implementation, a sample vessel loaded with a sample may be placed at the sample aspiration position by the operator, to enable the sample dispensing device 100 to aspirate the sample.

As an implementation, the reagent dispensing device 200 is configured to aspirate the reagent from the reagent vessel and dispense the aspirated reagent to the reaction vessel 20. The reagent vessel is configured to load the reagent. In this implementation, the reagent dispensing device 200 is used to aspirate the reagent from the reagent vessel and dispense the reagent to the reaction vessel 20, which is beneficial to achieve accurate quantification of the reagent.

As an implementation, the reagent dispensing device 200 includes a reagent needle, a second aspiration and discharge power device and a second movement power device. The second aspiration and discharge power device is connected to the reagent needle through a pipeline, to drive the reagent needle to perform aspiration and discharge actions, thereby achieving functions of aspirating and discharging the reagent by the reagent needle. The second movement power device is configured to drive the reagent needle to perform two-dimensional or three-dimensional spatial movement, to drive the reagent needle to move to different operation positions respectively, such as a reagent aspiration position, a reagent loading position, a cleaning position, a standby position of the reagent needle, etc. The reagent dispensing device 200 is configured to aspirate the reagent from a reagent vessel located at the reagent aspiration position and dispenses the reagent to a reaction vessel 20 located at the reagent loading position.

As an implementation, the sample analyzer 10 further includes a reagent storage device 800. The reagent storage device 800 is configured to store reagent vessels, and the reagent dispensing device 200 is configured to aspirate reagents from a reagent vessel in the reagent storage device 800 located at the reagent aspiration position. Of course, in a specific application, as an alternative implementation, the reagent storage device 800 may not be provided within the sample analyzer 10, for example, the reagent vessel is placed by the operator at the reagent aspiration position.

As an implementation, the reagent storage device 800 is of a disk shape, and the reagent storage device 800 is formed with multiple reagent positions distributed in a circumferential direction, each of the reagent positions is configured to accommodate a respective one of reagent vessels. Of course, in a specific application, the shape of the reagent storage device 800 is not limited thereto.

As an implementation, the cleaning liquid dispensing device 400 is provided with a function of heating the cleaning liquid and a function of cooling the cleaning liquid, that is, the cleaning liquid dispensing device 400 may output the heated cleaning liquid or may output the cooled cleaning liquid. In this implementation, the cleaning liquid dispensing device 400 is provided with a function of bidirectional temperature control for heating and cooling the cleaning liquid. Therefore, the cleaning liquid dispensing device 400 may supply the cleaning liquid within different temperature ranges according to different detection items and different reagents, so that the cleaning liquid may be controlled to be in an appropriate temperature zone under different application scenarios, such that the sample analyzer 10 may meet requirements of temperature-sensitive detection items better, particularly requirements of the cleaning liquid lower than ambient temperature in some detection items.

As an implementation, the sample analyzer 10 further includes a magnetic separation device 500, the cleaning liquid dispensing device 400 is configured to dispense the heated or cooled cleaning liquid in a target temperature range, to a reaction solution in the magnetic separation device 500 under control of the controller 102. That is, the cleaning liquid dispensing device 400 is configured to provide the reaction solution in the magnetic separation device 500 with the cleaning liquid that is required by a magnetic separation cleaning operation and reaches a temperature within the target temperature range. The magnetic separation device 500 performs the magnetic separation cleaning operation on a reaction solution in the reaction vessel 20 through the cleaning liquid provided by the cleaning liquid dispensing device 400 that is within the target temperature range. The sample detection device 300 is configured to detect the reaction solution prepared after the magnetic separation cleaning operation. The cleaning liquid supplied from the cleaning liquid dispensing device 400 to the reaction solution in the magnetic separation device 500, is also referred to as a separation liquid. The magnetic separation cleaning operation is mainly intended to clean off impurities in the reaction solution, while retaining to-be-detected parts, achieving purification of the reaction solution. The cleaning liquid supplied from the cleaning liquid dispensing device 400 to the magnetic separation device 500 is a cleaning liquid on which bidirectional temperature control may be performed, therefore on one hand, the magnetic separation device 500 may suit reagents with strict temperature-sensitive requirements better, enhancing capability of the sample analyzer 10 in suiting the reagents, reducing influence of ambient temperature on the temperature of the cleaning liquid, and improving clinical performance of temperature-sensitive items; on the other hand, compared to unidirectional temperature control, bidirectional temperature control may control the temperature of the cleaning liquid more accurately.

As an implementation, the reaction vessel 20 is at least configured to carry a sample and a reagent to generate an antigen-antibody binding reaction, to prepare a first reaction solution containing at least a first antigen-antibody binder. The magnetic separation device 500 is configured to perform a magnetic separation cleaning operation on the first reaction solution, to prepare a second reaction solution. The cleaning liquid dispensing device 400 is configured to provide the first reaction solution in the magnetic separation device 500 with the cleaning liquid that is required by the magnetic separation cleaning operation and reaches a temperature within the target temperature range. The sample detection device 300 configured to perform an optical signal detection on a third reaction solution prepared from the second reaction solution. The controller 102 is configured to analyze information of the optical signal detected by the sample detection device 300 to obtain the detection result of the sample. In this implementation, before the magnetic separation cleaning operation is performed, the first antigen-antibody binder is generated by the antigen-antibody binding reaction between the sample and the reagent, so that the first reaction solution configured to perform the magnetic separation cleaning operation contains at least the first antigen-antibody binder.

As an implementation, the reaction vessel 20 is at least configured to provide a reaction place for the sample and the reagent, so that a first reaction solution containing at least the first antigen-antibody binder is generated by an antigen-antibody binding reaction between a target antigen in the sample and an antibody in the reagent or between a target antibody in the sample and an antigen in the reagent. In this implementation, an aim of at least one sample detection item is to detect a content of the target antigen (a specific antigen) in the sample or a content of the target antibody (a specific antibody) in the sample. When an aim of a sample detection item is to detect the target antigen in the sample, the sample contains the target antigen, the reagent contains the antibody, and the first antigen-antibody binder is generated by the antigen-antibody binding reaction between the target antigen in the sample and the antibody in the reagent. When an aim of a sample detection item is to detect the target antibody in the sample, the sample contains the target antibody, the reagent contains the antigen, and the first antigen-antibody binder is generated by the antigen-antibody binding reaction between the target antibody in the sample and the antigen in the reagent. Various kinds of impurities are present in the sample, some impurities such as endogenous enzyme contained in a blood sample itself may affect a final detection system and accuracy of the detection result. Therefore, the detection solution used in this implementation includes a detection process as follows. First, the target antigen or the target antibody is purified; then, a level (i.e., content) of the target antigen or the target antibody in the sample is obtained through luminescence detection of a marker. The sample detection item with the antigen-antibody binding reaction is sensitive to temperature, and reagents with different components have different requirements on the temperature of the cleaning liquid. Therefore, in this implementation, the cleaning liquid supplied from the cleaning liquid dispensing device 400 to the magnetic separation device 500 may be the heated cleaning liquid or the cooled cleaning liquid, so that the cleaning liquid dispensed device 400 may supply the cleaning liquid to the magnetic separation device 500, such that the magnetic separation device 500 may suit reagents with strict temperature-sensitive requirements better, which meets requirements of development of low-temperature reagents, enhances capability of the sample analyzer 10 in suiting the reagents, and is beneficial to improve clinical performance of temperature-sensitive sample detection items; furthermore, compared to unidirectional temperature control, bidirectional temperature control may control the temperature of the cleaning liquid more accurately.

As an implementation, the sample analyzer 10 further includes a substrate dispensing device 101. The substrate dispensing device 101 is configured to dispense a luminescent substrate reagent to the second reaction solution prepared by the magnetic separation cleaning operation, to prepare a third reaction solution; the sample detection device 300 is configured to perform an optical signal detection on the third reaction solution.

As an implementation, the controller 102 is configured to control operations of the sample dispensing device 100, the reagent dispensing device 200, the sample detection device 300, the magnetic separation device 500, the substrate dispensing device 101 and the cleaning liquid dispensing device 400, and configured to analyze the optical signal information detected by the sample detection device 300 to obtain a content of the target antigen in the sample or a content of the target antibody in the sample.

As an implementation, the cleaning liquid dispensing device 400 includes a heat exchanger 410, a liquid path device 420 and a power device 430. The heat exchanger 410 is configured to perform heating and cooling respectively under control of the controller 102, the liquid path device 420 is configured to provide a flow path for a cleaning liquid, and at least a part of the liquid path device 420 is in contact with the heat exchanger 410, so as to allow the cleaning liquid in the liquid path device 420 to be heated or cooled by the heat exchanger 410 to reach a temperature within a target temperature range, the power device 430 is connected to the liquid path device 420 to drive the liquid path device 420 to aspirate a cleaning liquid from a cleaning liquid container 30 under control of the controller 102, and drive the liquid path device 420 to dispense the cleaning liquid that reaches a temperature within the target temperature range through heating or cooling by the heat exchanger 410, to the first reaction solution in the magnetic separation device 500 under control of the controller 102. In a specific application, a liquid path assembly may deliver the cleaning liquid to the heat exchanger 410 to exchange heat under driving of the power device 430, so that the cleaning liquid reaches a temperature within the target temperature range, and then dispense the cleaning liquid that is within the target temperature range to the first reaction solution in the magnetic separation device 500. It should be noted that contact between the liquid path device 420 and the heat exchanger 410 specifically means the contact between a component(s) of the liquid path device 420 and a component(s) of the heat exchanger 410, i.e., physical contact between structures, rather than thermal contact with a gap. In this way, it is beneficial to ensure heat exchange efficiency between the liquid path device 420 and the heat exchanger 410.

As an implementation, the heat exchanger includes a housing 415 and a heat exchange component 412 arranged in the housing 415, the liquid path device 420 includes a heat exchange tube section 4201, and the liquid path device 420 is at least partially arranged in the housing 415 and in contact with the heat exchange component 412. In this implementation, the liquid path device 420 is at least partially arranged in the housing 415 of the heat exchanger 410, and at least a part of the liquid path device 420 is located in the housing 415.

As an implementation, the heat exchanger includes a heat exchange component 412 and a single energy generation component 411. The single energy generation component 411 is configured to be switched between cooling and heating under control of the controller 102. The heat exchange component 412 is heat-conductive connected to the single energy generation component 411. The liquid path device 420 includes a heat exchange tube section 4201, the heat exchange tube section 4201 is in contact with the heat exchange component 412, so as to enable the cleaning liquid in the heat exchange tube section 4201 to exchange heat with the heat exchange component 412. In this implementation, the single energy generation component 411 is capable of performing both cooling and heating, that is, the component configured to perform cooling and the component configured to perform heating in the heat exchanger 410 are the same component. In this way, it is beneficial to simplify the structure of the heat exchanger 410.

As an implementation, the energy generation component 411 is a semiconductor cooling fin 4111. The semiconductor cooling fin 4111 is configured to be switched between cooling and heating under control of the controller 102. The semiconductor cooling fin 4111 is heat-conductive connected to the heat exchange component 412. The semiconductor cooling fin 4111 is also referred to as a thermoelectric cooling fin. In a specific application, cooling mode and heating mode of the semiconductor cooling fin 4111 may be changed by switching a direction of a current flowing into the semiconductor cooling fin 4111. In this implementation, the semiconductor cooling fin 4111 is used to perform cooling and heating respectively, which has advantages of simple structure, compact structure, high reliability, no refrigerant pollution, and good cooling and heating. Of course, in a specific application, as an alternative implementation, the semiconductor cooling fin 4111 may be specially configured to perform cooling, and an additional heater is provided to perform heating, that is, the heat exchanger 410 includes two energy generation components 411, i.e., the semiconductor cooling fin 4111 and the heater. Alternatively, as another alternative implementation, the semiconductor cooling fin 4111 may be specially configured to perform heating, and an additional cooler is provided to perform cooling, that is, the heat exchanger 410 includes two energy generation components 411, i.e., the semiconductor cooling fin 4111 and the cooler.

As an implementation, the heat exchange tube section 4201 is a tube section wound on or passes through the heat exchange component 412, and the heat exchange tube section 4201 is configured to carry the cleaning liquid to flow through the heat exchange component 412, so as to enable the cleaning liquid to exchange heat with the heat exchange component 412.

As an implementation, a groove 4121 is formed at a side of the heat exchange component 412, and the heat exchange tube section 4201 is at least partially embedded in the groove 4121 and in contact with an inner wall of the groove 4121. The heat exchange tube section 4201 is in contact with the heat exchange component 412 by contacting the inner wall of the groove 4121. In this way, on one hand, it is beneficial to achieve an aim of increasing a contact area between the heat exchange tube section 4201 and the heat exchange component 412 without increasing a size of the heat exchange component 412, therefore it is beneficial to increase a heat exchange effect between the cleaning liquid and the heat exchange component 412; on the other hand, it may be beneficial to improve reliability of positioning the heat exchange tube section 4201 on the heat exchange component 412. Of course, in a specific application, as an alternative implementation, the heat exchange component 412 may not be provided with the groove 4121 where the heat exchange tube section 4201 is embedded.

As an implementation, the heat exchange tube section 4201 is spirally wound on the heat exchange component 412. In this way, an aim of increasing the contact area between the heat exchange tube section 4201 and the heat exchange component 412 without increasing the size of the heat exchange component 412 may be achieved.

As an implementation, a spiral groove 4121 is formed at a side of the heat exchange component 412, and correspondingly, the heat exchange tube section 4201 is spirally wound on the heat exchange component 412.

As an implementation, the groove 4121 is formed at an outer side of the heat exchange component 412, and correspondingly, the heat exchange tube section 4201 is spirally wound on the outer side of the heat exchange component 412. In this way, it may facilitate assembly of the heat exchange tube section 4201 on the heat exchange component 412. Of course, in a specific application, as an alternative implementation, the heat exchange component 412 may be provided with a structure with a hollow inner hole, and the groove 4121 is provided at an inner side of the heat exchange component 412 (that is, the groove 4121 is recessed in an inner wall of the hollow inner hole).

As an implementation, the heat exchange tube section 4201 is wound on the heat exchange component 412, and the housing 415 surrounds respective outer circumference of the heat exchange tube section 4201 and the heat exchange component 412 to prevent heat of the heat exchange tube section 4201 and the heat exchange component 412 from dissipating to a surrounding environment. The housing 415 is made of a material with excellent heat insulation performance. The housing 415 is mainly intended to reduce loss of heat transfer between the heat exchange tube section 4201 and the heat exchange component 412. Of course, in a specific application, as an alternative implementation, the housing 415 may not be provided.

As an implementation, the heat exchanger 410 further includes a heat conductive block 413, the heat conductive block 413 is in contact with the semiconductor cooling fin 4111 and the heat exchange component 412 respectively to conduct heat of the semiconductor cooling fin 4111 to the heat exchange component 412. The heat conductive block 413 is made of a material with excellent heat conductivity. In this implementation, heat transfer between the semiconductor cooling fin 4111 and the heat exchange component 412 is achieved by using the heat conductive block 413, and the heat conductive block 413 is abutted against the semiconductor cooling fin 4111 and the heat exchange component 412 respectively, which may be beneficial to expand a heat exchange area through flexible structure design of the heat conductive block 413, therefore it is beneficial to increase heat transfer efficiency between the semiconductor cooling fin 4111 and the heat exchange component 412. Of course, in a specific application, as an alternative implementation, the heat conductive block 413 may not be provided, so that the semiconductor cooling fin 4111 is directly abutted against the heat exchange component 412 to transfer heat.

As an implementation, the heat exchanger 410 further includes a heat insulation component 414, the heat insulation component 414 surrounds an outer circumference of the heat conductive block 413 to prevent heat of the heat conductive block 413 from dissipating to the surrounding environment. The heat insulation component 414 is made of a material with excellent heat insulation performance. The heat insulation component 414 is mainly intended to reduce loss of heat transfer between the semiconductor cooling fin 4111 and the heat exchange component 412. Of course, in a specific application, as an alternative implementation, the heat insulation component 414 may not be provided.

As an implementation, the heat exchanger 410 further includes a heat sink 416, a side of the semiconductor cooling fin 4111 facing away from the heat conductive block 413 is abutted against the heat sink 416. The heat sink 416 is configured to exchange heat with the semiconductor cooling fin 4111.

As an implementation, the heat exchanger 410 further includes a heat dissipation fan 417, the heat dissipation fan 417 is arranged at a side of the heat sink 416 facing away from the semiconductor cooling fin 4111. In this implementation, heat of the semiconductor cooling fin 4111 is dissipated by using a solution of the heat sink 416 plus air cooling. Of course, in a specific application, as an alternative implementation, heat of the semiconductor cooling fin 4111 may be dissipated by using a solution of the heat sink 416 plus water cooling.

As an implementation, the cleaning liquid dispensing device 400 further includes a temperature detection component 440, the temperature detection component 440 is configured to detect a temperature of the heat exchange component 412 or detect a temperature of the cleaning liquid outputted through the heat exchange tube section 4201. The controller 102 is further configured to control operation of the semiconductor cooling fin 4111 according to temperature information fed back by the temperature detection component 440. The temperature detection component 440 is configured to detect the temperature of the heat exchange component 412 or the temperature of the cleaning liquid outputted from the heat exchange tube section 4201 in real time. The controller 102 controls the semiconductor cooling fin 4111 to perform cooling or heating according to feedback information of the temperature detection component 440, therefore it is beneficial to ensure that the cleaning liquid outputted from the heat exchange tube section 4201 may be maintained within a constant temperature range, and fully ensure accuracy and reliability of temperature control of the cleaning liquid.

As an implementation, controlling the operation of the semiconductor cooling fin 4111 according to the temperature information fed back by the temperature detection component 410 includes while performing a magnetic separation cleaning operation for a sample detection item: controlling the semiconductor cooling fin 4111 to perform cooling when the temperature fed back by the temperature detection component 440 is higher than an upper limit value of a target temperature range for the magnetic separation cleaning operation for the sample detection item, so that the heat exchange component 412 cools the cleaning liquid flowing through the heat exchange tube section 4201; and controlling the semiconductor cooling fin 4111 to perform heating when the temperature fed back by the temperature detection component 440 is lower than a lower limit value of the target temperature range for the magnetic separation cleaning operation for the sample detection item, so that the heat exchange component 412 heats the cleaning liquid flowing through the heat exchange tube section 4201. In this implementation, the temperature of the heat exchange component 412 or the temperature of the cleaning liquid outputted from the heat exchange tube section 4201 is controlled by the temperature information fed back by the temperature detection component 440, therefore it is beneficial to enable the temperature of the cleaning liquid outputted from the heat exchange tube section 4201 to be within a constant range. Specifically, in this implementation, an energy generation source of the bidirectional temperature control is provided by the semiconductor cooling fin 4111, and temperature of a bidirectional temperature control tank (i.e., the heat exchange component 412) is controlled to be constant according to a temperature sensor (i.e., the temperature detection component 440). When the temperature of the heat exchange component 412 is higher than control temperature (the upper limit value of the target temperature range), the semiconductor cooling fin 4111 performs cooling so that the temperature of the heat exchange component 412 is controlled to be within the target temperature range. When the temperature of the heat exchange component 412 is lower than the control temperature (the lower limit value of the target temperature range), the semiconductor cooling fin 4111 reversely heats the bidirectional temperature control tank to control its temperature to be within the target temperature range, thereby ensuring that the temperature of the heat exchange component 412 is always maintained in a constant temperature range under temperatures of different incoming flows and different environments, and correspondingly, an output temperature of the cleaning liquid is also maintained in a constant temperature range. Compared to unidirectional temperature control of heating, bidirectional temperature control of the cleaning liquid has at least advantages as follows: 1) Inclusiveness of the sample analyzer 10 to temperature control during development of reagents is improved, for example, consideration of temperature during development of magnetic bead reagents and marker reagents is reduced; 2) Signal-to-noise ratios of temperature-sensitive reagents are improved; 3) The problem of poor precision of liquid injection due to volatilization of the cleaning liquid at high temperature is solved.

As an implementation, the controller 102 is further configured to control a target temperature range for a magnetic separation cleaning operation for at least one sample detection item to be within 10°C to 35°C, that is, each temperature in the target temperature range for the at least one sample detection item is equal to or greater than 10°C and less than or equal to 35°C. The applicant found that when the temperature of the cleaning liquid is controlled to be within 10°C to 35°C, a phenomenon of dissociation of the antigen-antibody binder may be reduced. In related technologies prior to the application, the temperature of the cleaning liquid supplied to the magnetic separation device 500 is generally greater than ambient temperature, which easily induces the phenomenon that dissociation of the antigen-antibody binder in the reaction solution occurs, and causes the temperature of the cleaning liquid to be higher than ambient temperature. The cleaning liquid at a temperature higher than ambient temperature may affect sensitivity of the temperature-sensitive detection item, thereby affecting accuracy of the detection result. An appropriate temperature zone required by magnetic separation cleaning operations of some sample detection items is below ambient temperature, therefore an appropriate temperature zone of the cleaning liquid required by these sample detection items should also be below ambient temperature. In this implementation, it is ensured that the temperature of the cleaning liquid is not affected by ambient temperature mainly by performing bidirectional temperature control of the cleaning liquid, and the target temperature range may be controlled to be within 10°C to 35°C, to control the temperature of the cleaning liquid to be within an appropriate temperature zone, reduce the phenomenon of dissociation of the antigen-antibody binder, and effectively ensure accuracy of the sample detection result, which not only improves clinical performance of temperature-sensitive items, but also improves inclusiveness of the sample analyzer 10 to temperature during development of reagents, and it is beneficial to development of low-temperature reagents.

As an implementation, the controller 102 is further configured to control the target temperature range for the magnetic separation cleaning operation for the at least one sample detection item to withbe in a range of 15°C to 30°C, that is, each temperature in the target temperature range for the at least one sample detection item is within 15°C to 30°C, this temperature range is close to normal atmospheric temperature in a room, therefore it is more beneficial to improve clinical performance of temperature-sensitive items and inclusiveness of the sample analyzer 10 to development of low-temperature reagents.

As an implementation, the controller 102 is further configured to control the target temperature range for the magnetic separation cleaning operation for the at least one sample detection item to be within 24°C to 30°C, that is, each temperature in the target temperature range for the at least one sample detection item is within a range of 24°C to 30°C, this temperature range is close to normal atmospheric temperature in a room, therefore it is more beneficial to improve clinical performance of temperature-sensitive items and inclusiveness of the sample analyzer 10 to development of low-temperature reagents.

As an implementation, the controller 102 is further configured to control the target temperature range for the magnetic separation cleaning operation for the at least one sample detection item to be within 25°C to 29°C, that is, each temperature in the target temperature range for the at least one sample detection item is within 27°C±2°C.

As an implementation, the liquid path device 420 includes a main branch 422, a backflow branch 425, a liquid injection branch 423 and a switching device 424. One end of the main branch 422 is configured to aspirate the cleaning liquid from the cleaning liquid container 30, the other end of the main branch 422 is connected to the switching device 424 that switches between the backflow branch 425 and the liquid injection branch 423, and at least a part of the main branch 422 forms a heat exchange tube section 4201 in contact with the heat exchanger 410, so as to allow the cleaning liquid in the heat exchange tube section 4201 to be heated or cooled by the heat exchanger 410. The power device 430 is connected to the main branch 422; the liquid injection branch 423 is configured to dispense the cleaning liquid; the backflow branch 425 forms a circulation loop with the main branch 422 and the cleaning liquid container 30, or the backflow branch 425 forms a circulation loop with the main branch 422, so that the cleaning liquid outputted from the main branch 422 is guided by the backflow branch 425 to circulate through the heat exchanger 410, so as to exchange heat. The cleaning liquid container 30 is configured to store the cleaning liquid. In this implementation, the cleaning liquid is circulated by providing the circulation loop, so that the cleaning liquid may circulate through the heat exchanger 410 to exchange heat, and after the temperature of the cleaning liquid in the circulation loop is within the target temperature range, the cleaning liquid is output to the first reaction solution of the magnetic separation device 500, thereby fully ensuring that the cleaning liquid outputted from the liquid path device 420 may meet temperature control requirements.

As an implementation, the main branch 422, the backflow branch 425 and the cleaning liquid container 30 are connected end-to-end in sequence, to form a circulation loop. That is, an input end of the backflow branch 425 is connected to an output end of the main branch 422, an output end of the backflow branch 425 and an input end of the main branch 422 are connected to the cleaning liquid container 30 respectively, the backflow branch 425 delivers the back-flowed cleaning liquid to the cleaning liquid container 30, then the cleaning liquid flows from the cleaning liquid container 30 to the main branch 422, and circulates through the heat exchanger 410 to exchange heat. Of course, in a specific application, as an alternative implementation, the main branch 422 may be connected to the backflow branch 425 separately to form a circulation loop, that is, the input end of the backflow branch 425 is connected to the output end of the main branch 422, and the output end of the backflow branch 425 is connected to the input end of the main branch 422. In this alternative implementation, the cleaning liquid flowed back through the backflow branch 425 directly flows back to the main branch 422 without passing through the cleaning liquid container 30, and circulates through the heat exchanger 410 to exchange heat.

As an implementation, a liquid path formed by the liquid injection branch 423 has a length less than or equal to 200 mm. In this way, a liquid path of a non-backflow part of the cleaning liquid dispensing device 400 has a length less than or equal to 200 mm, therefore it is beneficial to result in a small amount of a non-backflow cleaning liquid, and thus it is beneficial to ensure accuracy and reliability of the temperature of the cleaning liquid outputted from the cleaning liquid dispensing device 400 each time.

As an implementation, the liquid path formed by the liquid injection branch 423 has a length less than or equal to 20 mm. In this implementation, accuracy and reliability of the temperature of the cleaning liquid outputted from the cleaning liquid dispensing device 400 each time are ensured to a great extent by arranging the length of the liquid path of the non-backflow part of the cleaning liquid dispensing device 400 to be within 20 mm.

As an implementation, a volume of the liquid injection branch 423 is less than or equal to 10% of a liquid amount of the cleaning liquid that is dispensed by the liquid injection branch 423 into the first reaction solution in the magnetic separation device 500 at one time. In this implementation, an aim of reducing an amount of the cleaning liquid in the non-backflow part of the cleaning liquid dispensing device 400 may also be achieved by limiting the volume of the liquid injection branch 423, therefore it is also beneficial to ensure accuracy and reliability of the temperature of the cleaning liquid outputted from the cleaning liquid dispensing device 400 each time.

As an implementation, a volume of the liquid injection branch 423 is less than or equal to 5% of the liquid amount of the cleaning liquid that is dispensed by the liquid injection branch 423 into the first reaction solution in the magnetic separation device 500 at one time. In this implementation, further limiting the volume of the liquid injection branch 423 is beneficial to reduce the amount of the cleaning liquid in the non-backflow part of the cleaning liquid dispensing device 400 better.

As an implementation, the switching device 424 is formed with a liquid inlet *a*, a backflow port *c* and a liquid outlet *b.* The liquid inlet *a* is configured to enable the cleaning liquid after exchanging heat through the heat exchanger 410 to flow into the switching device 424. The backflow port *c* is configured to enable the cleaning liquid flowing into the switching device 424 to flow back to the main branch 422 through the backflow branch 425, so as to exchange heat with the heat exchanger 410. The liquid outlet b is configured to output the cleaning liquid to the liquid injection branch 423.

As an implementation, the liquid path device 420 further includes a confluence component 421, the switching device 424 is mounted on the confluence component 421, the liquid outlet *b* is abutted against the confluence component 421. The confluence component 421 mainly plays a function of confluence, and a channel enabling the cleaning liquid to flow is formed inside the confluence component 421. The confluence component 421 plays a function of delivering the cleaning liquid through a rigid structure instead of a pipeline, has a compact structure and is convenient for installation. In this implementation, the switching device 424 is directly mounted on the confluence component 421, so that on one hand, a mounting bracket of the switching device 424 may be omitted; on the other hand, a pipeline between the switching device 424 and the confluence component 421 may be omitted, further simplifying structure of the sample analyzer 10.

As an implementation, the switching device 424 is a multi-way valve, the multi-way valve is provided with at least two operation positions and at least three interfaces, and the liquid inlet *a*, the backflow port c and the liquid outlet *b* are three interfaces of the multi-way valve respectively. Of course, in a specific application, arrangement of the switching device 424 is not limited thereto. For example, at least two two-position two-way valves may be used instead.

As an implementation, the switching device 424 is a two-position three-way valve, and the liquid inlet *a*, the backflow port c and the liquid outlet *b* are three interfaces of the two-position three-way valve respectively. The two-position three-way valve is provided with two operation positions: at the first operation position, the liquid inlet *a* is communicated with the backflow port *c,* and the cleaning liquid entering the switching device 424 flows back to the main branch 422 through the backflow branch 425, so as to exchange heat with the heat exchanger 410; at the second operation position, the liquid inlet *a* is communicated with the liquid outlet, and the cleaning liquid entering the switching device 424 is discharged from the liquid injection branch 423 and dispensed to the first reaction solution in the magnetic separation device 500. Of course, in a specific application, arrangement of the switching device 424 is not limited thereto. For example, two two-position two-way valves may be used instead.

As an implementation, the backflow port *c* is also abutted against the confluence component 421, the backflow branch 425 includes a backflow guide channel 4252 formed in the confluence component 421 and a backflow pipeline 4251 at least partially arranged outside the confluence component 421, two ends of the backflow guide channel 4252 are communicated with an input end of the backflow pipeline 4251 and the backflow port c respectively, so that the cleaning liquid flowing out of the backflow port c flows to the backflow pipeline 4251 through the backflow guide channel 4252, and an output end of the backflow pipeline 4251 is communicated with the cleaning liquid container 30. A part of the backflow branch 425 (i.e. the backflow guide channel 4252) is formed by the confluence component 421, so that a pipeline connecting the backflow branch 425 to the switching device 424 may be reduced. In this implementation, the output end of the backflow pipeline 4251 is communicated with the cleaning liquid container 30, and the main branch 422, the backflow guide channel 4252, the backflow pipeline 4251 and the cleaning liquid container 30 are connected end-to-end in sequence, to form a circulation loop. That is, the input end of the backflow branch 425 is connected to the output end of the main branch 422, the output end of the backflow branch 425 and the input end of the main branch 422 are connected to the cleaning liquid container 30 respectively, the backflow branch 425 delivers the back-flowed cleaning liquid to the cleaning liquid container 30, then the cleaning liquid flows from the cleaning liquid container 30 to the main branch 422, and circulates through the heat exchanger 410 to exchange heat. Of course, in a specific application, as an alternative implementation, the output end of the backflow pipeline 4251 may be communicated with the main branch 422, and the main branch 422 is connected to the backflow branch 425 separately to form a circulation loop, that is, the input end of the backflow branch 425 is connected to the output end of the main branch 422, and the output end of the backflow branch 425 is connected to the input end of the main branch 422.

As an implementation, the liquid inlet *a* is also abutted against the confluence component 421, the main branch 422 includes a liquid inlet guide channel 4222 formed in the confluence component 421 and a main liquid delivery pipeline 4221 arranged outside the confluence component 421, the main liquid delivery pipeline 4221 extends from the cleaning liquid container 30 to the liquid inlet guide channel 4222, at least a part of the main liquid delivery pipeline 4221 forms the heat exchange tube section 4201, two ends of the liquid inlet guide channel 4222 are communicated with an output end of the main liquid delivery pipeline 4221 and the liquid inlet *a* respectively, so that the cleaning liquid flowing out of the main liquid delivery pipeline 4221 flows to the switching device 424 through the liquid inlet guide channel 4222. In this implementation, a part of the main branch 422 (i.e., the liquid inlet guide channel 4222) is formed by the confluence component 421, and a pipeline connecting the main branch 422 to the switching device 424 may be reduced.

As an implementation, the liquid injection branch 423 includes a liquid injection guide channel 4232 formed in the confluence component 421, and a first liquid injection component 4231 at least partially arranged outside the confluence component 421 to dispense the cleaning liquid. Two ends of the liquid injection guide channel 4232 are communicated with the liquid outlet *b* and the first liquid injection component 4231 respectively, that is, the liquid injection guide channel 4232 is connected between the liquid outlet *b* and the first liquid injection component 4231. The first liquid injection component 4231 is configured to inject the cleaning liquid outputted from the cleaning liquid dispensing device 400 within the target temperature range into the first reaction solution in the magnetic separation device 500. In this implementation, a part of the liquid injection branch 423 (i.e., the liquid injection guide channel 4232) is formed by the confluence component 421, so that the pipeline connecting the main branch 422 to the switching device 424 can be reduced. In a specific application, after the cleaning liquid in the circulation loop reaches a temperature within the target temperature range, the cleaning liquid is output to the liquid injection guide channel 4232 and the first liquid injection component 4231, to fully ensure that the cleaning liquid outputted from the first liquid injection component 4231 can meet temperature control requirements.

As an implementation, the first liquid injection component 4231 is directly connected to the liquid injection guide channel 4232, and a length of the liquid path formed by the liquid injection branch 423 is a sum of a length of a liquid path of the liquid injection guide channel 4232 and a length of a liquid path of the first liquid injection component 4231. Correspondingly, the liquid path formed by the liquid injection branch 423 has a length less than or equal to 200 mm, that is, the sum of the length of the liquid path of the liquid injection guide channel 4232 and the length of the liquid path of the first liquid injection component 4231 is less than or equal to 200 mm. The liquid path formed by the liquid injection branch 423 has a length less than or equal to 20 mm, that is, the sum of the length of the liquid path of the liquid injection guide channel 4232 and the length of the liquid path of the first liquid injection component 4231 is less than or equal to 20 mm. In this implementation, the first liquid injection component 4231 is directly connected to the liquid injection guide channel 4232. Of course, in a specific application, as an alternative implementation, the first liquid injection component 4231 may be indirectly connected to the liquid injection guide channel 4232 through a pipeline, and the length of the liquid path formed by the liquid injection branch 423 is a sum of the length of the liquid path of the liquid injection guide channel 4232, the length of the liquid path of the first liquid injection component 4231, and a length of the pipeline connected between the liquid injection guide channel 4232 and the first liquid injection component 4231.

As an implementation, the first liquid injection component 4231 is directly connected to the liquid injection guide channel 4232, and a volume of the liquid injection branch 423 is a sum of a volume of the liquid injection guide channel 4232 and a volume of the first liquid injection component 4231. Correspondingly, a volume of the liquid injection branch 423 is less than or equal to 10% of the liquid amount of the cleaning liquid that is dispensed by the liquid injection branch 423 into the first reaction solution in the magnetic separation device 500 at one time, that is, the sum of the volume of the liquid injection guide channel 4232 and the volume of the first liquid injection component 4231 is less than or equal to 10% of the liquid amount of the cleaning liquid that is dispensed by the first liquid injection component 4231 into the first reaction solution in the magnetic separation device 500 at one time. A volume of the liquid injection branch 423 is less than or equal to 5% of the liquid amount of the cleaning liquid that is dispensed by the liquid injection branch 423 into the first reaction solution in the magnetic separation device 500 at one time, that is, the sum of the volume of the liquid injection guide channel 4232 and the volume of the first liquid injection component 4231 is less than or equal to 5% of the liquid amount of the cleaning liquid that is dispensed by the first liquid injection component 4231 into the first reaction solution in the magnetic separation device 500 at one time. In this implementation, the first liquid injection component 4231 is directly connected to the liquid injection guide channel 4232. Of course, in a specific application, as an alternative implementation, the first liquid injection component 4231 may be indirectly connected to the liquid injection guide channel 4232 through a pipeline, and a volume of the liquid injection branch 423 is a sum of the volume of the liquid injection guide channel 4232, the volume of the first liquid injection component 4231, and a volume of the pipeline connected between the liquid injection guide channel 4232 and the first liquid injection component 4231.

As an implementation, a liquid injection port is formed at an end of the first liquid injection component 4231 away from the switching device 424. A length of a liquid path formed by the liquid injection branch 423 and the first liquid injection component 4231 is a length of a liquid path between the liquid injection port and the liquid outlet *b.* A sum of a volume of the liquid injection branch 423 and a volume of the first liquid injection component 4231 is a volume of the liquid path between the liquid injection port and the liquid outlet *b.*

As an implementation, the first liquid injection component 4231 penetrates into the confluence component 421 to dispense the cleaning liquid that reaches a temperature within the target temperature range through heating or cooling by the heat exchanger 410 and that is delivered to the confluence component 421, into the first reaction solution located in the magnetic separation device 500. In this implementation, the first liquid injection component 4231 directly penetrates into the confluence component 421, so that on one hand, the first liquid injection component 4231 may be mounted without using a separate bracket, therefore it is beneficial to simplify structure of mounting the first liquid injection component 4231; on the other hand, it is beneficial to reduce delivery pipeline of the cleaning liquid. The main branch 422 may deliver the cleaning liquid to the heat exchanger 410 to exchange heat, so that the cleaning liquid reaches a temperature within the target temperature range, and then the cleaning liquid that is within the target temperature range is delivered to the first liquid injection component 4231. Of course, in a specific application, as an alternative implementation, the first liquid injection component 4231 may not penetrate into the confluence component 421, instead, may be arranged to be connected to the confluence component 421 through a pipeline. In this alternative implementation, the length of the liquid path formed by the liquid injection branch 423 is a sum of the length of the liquid path of the liquid injection guide channel 4232, the length of the liquid path of the first liquid injection component 4231, and a length of the pipeline connected between the liquid injection guide channel and the first liquid injection component 4231, and a volume of the liquid injection branch 423 is a sum of the volume of the liquid injection guide channel 4232, the volume of the first liquid injection component 4231, and a volume of the pipeline connected between the liquid injection guide channel and the first liquid injection component 4231.

As an implementation, the first liquid injection component 4231 is a liquid injection needle, that is, the first liquid injection component 4231 is a needle-shaped component. Of course, in a specific application, as an alternative implementation, the first liquid injection component 4231 may be a liquid injection tube.

As an implementation, the first liquid injection component 4231 is arranged obliquely relative to a vertical direction, that is, the first liquid injection component 4231 does not extend in the vertical direction.

As an implementation, the confluence component 421 is a block-shaped component, that is, the confluence component 421 is a confluence block, and the liquid inlet guide channel 4222, the backflow guide channel 4252 and the liquid injection guide channel 4232 are all formed in the confluence block, that is, the liquid inlet guide channel 4222, the backflow guide channel 4252 and the liquid injection guide channel 4232 are flow channels integrally formed in the confluence block. The liquid inlet guide channel 4222, the backflow guide channel 4252 and the liquid injection guide channel 4232 are microporous flow channels in the confluence block, and among these parts through which backflow does not pass, only the microporous flow channel part of the liquid injection guide channel 4232 can be subjected to temperature control by a heat exchange element on the confluence component 421, thereby improving accuracy. Of course, in a specific application, the liquid inlet guide channel 4222, the backflow guide channel 4252 and the liquid injection guide channel 4232 may be formed in form of buried pipes in the confluence block.

As an implementation, sealing rings are arranged at docking positions of interfaces of the switching device 424 (i.e., the liquid inlet *a*, the backflow port *c* and the liquid outlet *b*) and corresponding flow channels on the confluence component 421 (i.e., the liquid inlet guide channel 4222, the backflow guide channel 4252 and the liquid injection guide channel 4232), and the switching device 424 is connected to the confluence component 421 by screws or bolts to compress each sealing ring tightly, thereby avoiding liquid leakage and liquid permeation while ensuring connection.

As an implementation, an orthographic projection of the liquid outlet *b* on the confluence component 421 does not overlap with an orthographic projection of the first liquid injection component 4231 on the confluence component 421, that is, the orthographic projection of the liquid outlet *b* on the confluence component 421 is staggered from the orthographic projection of the first liquid injection component 4231 on the confluence component 421. The liquid injection guide channel 4232 includes a first channel part 4207 and a second channel part 4208, one end of the first channel part 4207 is communicated with the liquid outlet *b,* the second channel part 4208 extends from the other end of the first channel part 4207 to the first liquid injection component 4231, and an angle greater than 0° and less than 180° is formed at a junction of the first channel part 4207 and the second channel part 4208. In this implementation, the first liquid injection component 4231 is not directly connected to the liquid outlet *b.* In this way, it is beneficial to achieve a more flexible angle of mounting the first liquid injection component 4231 on the confluence component 421.

As an implementation, an orthographic projection of the liquid outlet *b* on the confluence component 421 does not overlap with an orthographic projection of the first liquid injection component 4231 on the confluence component 421, that is, the orthographic projection of the liquid outlet *b* on the confluence component 421 and the orthographic projection of the first liquid injection component 4231 on the confluence component 421 do not overlap with each other. In this implementation, the orthographic projection of the liquid outlet *b* on the confluence component 421 is completely staggered from the orthographic projection of the first liquid injection component 4231 on the confluence component 421, that is, an interval is present between the liquid outlet *b* and the first liquid injection component 4231 in a horizontal direction.

As an implementation, an angle of 90°±10° is formed at the junction of the first channel part 4207 and the second channel part 4208, that is, the first channel part 4207 is substantially perpendicular to the second channel part 4208.

As an implementation, the first channel part 4207 is arranged to extend in the vertical direction, and the second channel part 4208 is arranged to extend in the horizontal direction.

As an implementation, the cleaning liquid dispensing device 400 further includes a heat exchange element (not shown in the figures), the heat exchange element is arranged within the confluence component 421 and adjacent to the liquid injection guide channel 4232, to exchange heat with the cleaning liquid flowing through the liquid injection guide channel 4232. In this implementation, the heat exchange element is provided to exchange heat with the cleaning liquid flowing through the liquid injection guide channel 4232. In this way, it is beneficial to compensate heat loss generated by the cleaning liquid flowing through the liquid injection guide channel 4232. Of course, in a specific application, as an alternative implementation, the heat exchange element may not be provided, especially when the liquid injection guide channel 4232 is short enough.

As an implementation, the heat exchange element is configured to heat and/or cool the cleaning liquid flowing through the liquid injection guide channel 4232. The heat exchange element may be provided with both a heating function and a cooling function; or, the heat exchange element may be only provided with the heating function; or, the heat exchange element may be only provided with the cooling function. Specifically, when the heat exchange element is provided with both the heating function and the cooling function, the heat exchange element may heat the cleaning liquid flowing through the liquid injection branch 423 under control of the controller 102, or may cool the cleaning liquid flowing through the liquid injection branch 423 under control of the controller 102. When the heat exchange element is only provided with the heating function, the heat exchange element may only heat the cleaning liquid flowing through the liquid injection branch 423 under control of the controller 102. When the heat exchange element is only provided with the cooling function, the heat exchange element may only cool the cleaning liquid flowing through the liquid injection branch 423 under control of the controller 102.

As an implementation, the heat exchange element is arranged close to the second channel part 4208, to exchange heat with the cleaning liquid flowing through the second channel part 4208. In this implementation, the heat exchange element is provided to exchange heat with the cleaning liquid flowing through the second channel part 4208. Of course, in a specific application, as an alternative implementation, the heat exchange element may be arranged close to the first channel part 4207.

As an implementation, the circulation loop further includes a liquid inlet valve 426 and a liquid injection valve 427. The liquid inlet valve 426 is connected between the cleaning liquid container 30 and the power device 430 through a pipeline, to control on and off of the pipeline between the cleaning liquid container 30 and the power device 430. The liquid injection valve 427 is connected between the heat exchanger 410 and the power device 430 through a pipeline, to control on and off of the pipeline between the heat exchanger 410 and the power device 430. An end of the backflow branch 425 and an end of the main branch 422 are connected to the cleaning liquid container 30 respectively.

As an implementation, the main branch 422 includes a heat exchange tube section 4201, a first tube 4202, a second tube 4203, a third tube 4204, a fourth tube 4205 and a fifth tube 4206. The heat exchange tube section 4201 is configured to carry the cleaning liquid to flow through the heat exchanger 410, so as to exchange heat, the first tube 4202 is connected between one end of the heat exchange tube section 4201 and the liquid inlet guide channel 4222 of the confluence component 421, the second tube 4203 is connected between the other end of the heat exchange tube section 4201 and the liquid injection valve 427, the third tube 4204 is connected between the liquid injection valve 427 and the power device 430, the fourth tube 4205 is connected between the power device 430 and the liquid inlet valve 426, the fifth tube 4206 is connected between the liquid inlet valve 426 and the cleaning liquid container 30, and the backflow pipeline 4251 is connected between the cleaning liquid container 30 and the backflow guide channel 4252 of the confluence component 421. The heat exchange tube section 4201, the first tube 4202, the liquid inlet guide channel 4222, the switching device 424, the backflow guide channel 4252, the backflow pipeline 4251, the cleaning liquid container 30, the fifth tube 4206, the liquid inlet valve 426, the fourth tube 4205, the power device 430, the third tube 4204, the liquid injection valve 427 and the second tube 4203 are connected end-to-end in sequence, to form a circulation loop.

As an implementation, both the liquid inlet valve 426 and the liquid injection valve 427 are two-position two-way valves.

As an implementation, the power device 430 includes a syringe, the syringe may quantify an amount of the cleaning liquid accurately. Of course, in a specific application, arrangement of the power device 430 is not limited thereto. For example, as an alternative implementation, the power device 430 may be a pump.

As an implementation, the sample analyzer 10 further includes a liquid aspiration assembly 530, the aspiration assembly 530 includes a liquid aspiration component 531 and a liquid aspiration power device. The liquid aspiration component 531 penetrates into the confluence component 421 and is fixed to the confluence component 421, to aspirate a clear liquid in the second reaction solution after the magnetic separation cleaning operation under driving of the liquid aspiration power device.

As an implementation, the liquid aspiration component 531 is arranged in the vertical direction, and the first liquid injection component 4231 is arranged obliquely relative to the vertical direction, with a trend of its bottom end gradually approaching the liquid aspiration component 531.

As an implementation, the magnetic separation device 500 includes a magnetic separation disk 510 and a lid 520. The magnetic separation disk 510 is configured to accommodate multiple reaction vessels 520. The lid 520 covers the magnetic separation disk 510. The liquid path device 420 further includes a main branch 422, a confluence component 421 and a liquid injection branch 423. The liquid injection branch 423 includes a liquid injection guide channel 4232 formed in the confluence component 421 and a first liquid injection component 4231 at least partially arranged outside the confluence component 421 to dispense the cleaning liquid. The main branch 422 is communicated with the first liquid injection component 4231 through the confluence component 421. The confluence component 421 is mounted on the lid 520. In this implementation, the confluence component 421 is directly mounted on the lid 520 of the magnetic separation device 500, and the confluence component 421, the first liquid injection component 4231 and pipeline are mounted without providing an additional bracket, thereby simplifying structure of the sample analyzer 10 to a great extent.

As an implementation, the cleaning liquid with different temperatures required by different detection items is dispensed through the first liquid injection component 4231. Of course, in a specific application, as an alternative implementation, a second liquid injection component independent of the first liquid injection component 4231 may be provided separately, to dispense the cleaning liquid on which temperature control is not required. For example, the cleaning liquid dispensing device 400 further includes a second liquid injection component and a heat insulation material layer. The second liquid injection component is configured to dispense the cleaning liquid that is within the target temperature range to the reaction vessel 20 in the magnetic separation device 500. An end of the second liquid injection component penetrates into the confluence component 421 and is fixed to the confluence component 421, and the second liquid injection component is arranged at an interval from the first liquid injection component 4231. The heat insulation material layer is arranged in the confluence component 421 and between the first liquid injection component 4231 and the second liquid injection component, to prevent heat conduction between the first liquid injection component 4231 and the second liquid injection component.

As an implementation, the controller 102 is further configured to adjust a sequence of multiple sample detection items performing their respective magnetic separation cleaning operations, according to different temperature control requirements of multiple sample detection items. Since different sample detection items have different temperature control requirements, it may be beneficial to ensure detection efficiency of a batch of samples, and may be beneficial to reduce influence of the temperature of the cleaning liquid on the detection result of each sample detection item, by adjusting the sequence of different sample detection items.

As an implementation, the controller 102 is further configured to control the magnetic separation device 500 to perform a magnetic separation cleaning operation on each of one or more detection items of a first type and then control the magnetic separation device 500 to perform a magnetic separation cleaning operation on each of one or more detection items of a second type when multiple sample detection items for a same batch of samples are to be performed; the one or more detection items of the first type and the one or more detection items of the second type meet at least one of following conditions: a temperature control requirement of the one or more detection items of the first type is lower than that of the one or more detection items of the second type; or a difference between a target temperature range required to perform the magnetic separation cleaning operation on each of the one or more detection items of the first type and ambient temperature is smaller than a difference between a target temperature range required to perform the magnetic separation cleaning operation on each of one or more detection items of the second type and ambient temperature. In this implementation, temperature-sensitive detection items or detection items with a large difference between the target temperature range and ambient temperature are arranged behind other detection items, to enable other detection items to use the cleaning liquid close to ambient temperature first, which is beneficial to balance detection efficiency of the batch of samples and accuracy of detection results of sample detection items well.

As an implementation, the controller 102 is further configured to configure different temperature control parameters for at least two sample detection items according to different sample detection items and/or different liquid injection amounts of the cleaning liquid. Here, the different temperature control parameters include at least one of different parameters as follows: operation power of the heat exchanger 410, a liquid amount of the cleaning liquid flowing back through a backflow branch 425 before dispensing the cleaning liquid to the first reaction solution while performing a single sample detection item, or a liquid amount of the cleaning liquid flowing through the heat exchanger 410 before dispensing the cleaning liquid to the first reaction solution while performing the single sample detection item. Operation power of the heat exchanger 410 is adjusted, so that heat exchange efficiency may be ensured. While performing a single sample detection item, the larger the amount of the cleaning liquid that flows back, the more stable the temperature of the cleaning liquid. In this implementation, different temperature control parameters of the cleaning liquid are configured for different sample detection items and/or different liquid injection amounts of the cleaning liquid, which is beneficial to balance detection efficiency and accuracy of detection results.

As an implementation, with respect to different sample detection items and/or different liquid injection amounts of the cleaning liquid, at least the following temperature control parameters of the cleaning liquid are configured differently: heating power, and a backflow amount of the cleaning liquid during a single sample detection.

As an implementation, the controller 102 is further configured to configure different temperature control parameters for at least two sample detection items according to different ambient temperatures. Here, the different temperature control parameters include at least one of different parameters as follows: operation power of the heat exchanger 410, a liquid amount of the cleaning liquid flowing back through a backflow branch 425 before dispensing the cleaning liquid to the first reaction solution of a first sample detection item while performing multiple sample detection items for a same batch of samples, a liquid amount of the cleaning liquid flowing through the heat exchanger 410 before dispensing the cleaning liquid to the first reaction solution of the first sample detection item while performing multiple sample detection items for the same batch of samples, a liquid amount of the cleaning liquid flowing through the backflow branch 425 while performing multiple sample detection items for the same batch of samples, or a frequency of the cleaning liquid flowing through the backflow branch 425 while performing multiple sample detection items for the same batch of samples. In this implementation, configuring different temperature control parameters of the cleaning liquid for different ambient temperatures is beneficial to balance detection efficiency and accuracy of detection results.

As an implementation, with respect to different ambient temperatures, at least the following temperature control parameters of the cleaning liquid are configured differently: heating power, a total backflow amount of the cleaning liquid before the batch, a backflow frequency of the cleaning liquid in batch, and a backflow amount of the cleaning liquid in batch.

As an implementation, the magnetic separation device 500 is configured to perform magnetic attraction on the reaction solution in the reaction vessel 20, to separate the reaction solution in the reaction vessel 20 to form a magnetic bead liquid and a clear liquid, the clear liquid is treated as a waste liquid, and the magnetic bead liquid is configured to prepare a to-be-detected liquid for subsequent sample detection items.

As an implementation, the magnetic separation device 500 includes a holding assembly, a transfer assembly, at least one liquid aspiration assembly 530, at least one first magnetic assembly 540 and at least one second magnetic assembly 550. The transfer assembly is at least configured to transfer a reaction vessel 20 loaded with the first reaction solution to a first magnetic attraction position 501, and transfer a reaction vessel 20 after completing the first magnetic attraction action to a first liquid aspiration position 502. The holding assembly is at least configured to hold the reaction vessel 20 located at the first magnetic attraction position 501 and hold the reaction vessel 20 located at the first liquid aspiration position 502. The first magnetic assembly 540 is configured to perform a first magnetic attraction action on the first reaction solution in a reaction vessel 20 located at the first magnetic attraction position 501. The second magnetic assembly 550 is configured to perform a second magnetic attraction action on the first reaction solution in a reaction vessel 20 located at the first liquid aspiration position 502. The liquid aspiration assembly 530 is configured to perform a first liquid aspiration action on the first reaction solution in the reaction vessel 20 located at the first liquid aspiration position 502. The magnetic attraction action at the first magnetic attraction position 501 and the liquid aspiration action at the first liquid aspiration position 502 may be performed in parallel, therefore it is beneficial to ensure magnetic separation efficiency of the batch of samples. The magnetic attraction action at the first magnetic attraction position 501 is mainly intended to attract magnetic beads in the reaction vessel 20 and a target substance combined with the magnetic beads (here, the target substance may be a to-be-detected substance or an interference substance) to a designated position as quick as possible, to form a magnetic bead group 40 on an inner wall of the reaction vessel 20, and the magnetic attraction action at the first liquid aspiration position 502 is mainly intended to attract magnetic beads in the reaction vessel 20 and the target substance combined with the magnetic beads on the inner wall of the reaction vessel 20 as stabe as possible.

As an implementation, the first magnetic attraction position 501 is configured to accommodate the reaction vessel 20 to perform the first magnetic attraction action while not performing the liquid aspiration action, and the first liquid aspiration position 502 is configured to accommodate the reaction vessel 20 to perform the second magnetic attraction action and the first liquid aspiration action.

As an implementation, the first magnetic assembly 540 forms at least two spaced magnetic bead groups 40 on an inner side wall of the reaction vessel 20 located at the first magnetic attraction position 501 by attraction; and the second magnetic assembly 550 forms at least one magnetic bead group 40 on an inner side wall of the reaction vessel 20 located at the first liquid aspiration position 502 by attraction. The liquid aspiration assembly 530 is configured to perform a first liquid aspiration action on the first reaction solution in the reaction vessel 20 located at the first liquid aspiration position 502 and subjected to the second magnetic attraction action performed by the first magnetic assembly 540. The number of magnetic bead groups 40 formed by the first magnetic assembly 540 attracting on the inner side wall of the reaction vessel 20 located at the first magnetic attraction position 501 is greater than the number of magnetic bead groups 40 formed by the second magnetic assembly 550 attracting on the inner side wall of the reaction vessel 20 located at the first liquid aspiration position 502. The first magnetic assembly 540 forms at least two spaced magnetic bead groups 40 on the inner side wall of the reaction vessel 20 located at the first magnetic attraction position 501 by the attraction, mainly because the first magnetic assembly 540 forms at least two dispersed magnetic fields at the first magnetic attraction position 501. The at least two dispersed magnetic fields may attract, from different directions, magnetic beads in the reaction vessel 20 located at the first magnetic attraction position 501 and the target substance combined with the magnetic beads, so that it may shorten distances moved by the magnetic beads and substances combined with the magnetic beads when they are attracted, and the magnetic beads and substances combined with the magnetic beads may be quickly attracted to the inner side wall of the reaction vessel 20 nearby, which is beneficial to ensure attraction speed at the first magnetic attraction position 501. In this implementation, the first magnetic assembly 540 located at the first magnetic attraction position 501 and the second magnetic assembly 550 located at the first liquid aspiration position 502 are arranged to have different modes, and the number of magnetic bead groups 40 formed by attraction of the first magnetic assembly 540 in a reaction vessel 20 is greater than a number of magnetic bead groups 40 formed by attraction of the second magnetic assembly 550 in a reaction vessel 20, so that it is beneficial to ensure both the attraction speed at the first magnetic attraction position 501 and attraction stability at the first liquid aspiration position 502. Furthermore, in the magnetic separation cleaning operation, the longer the magnetic attraction time, the larger the amount of dissociation of the antigen-antibody binder. Therefore, in this implementation, at least two spaced magnetic bead groups 40 formed by attraction of the first magnetic assembly 540 may quickly attract the magnetic beads, thereby achieving quick magnetic separation of the clear liquid and the magnetic bead liquid, which can further reduce the phenomenon of dissociation of the antigen-antibody binder, and can be beneficial to solve the dissociation problem of temperature-sensitive items better, with cooperation of bidirectional temperature control of the cleaning liquid.

As an implementation, the first magnetic attraction position 501 is formed in between first magnetic assembly 540 or at a side of the first magnetic assembly 540, that is, the first magnetic assembly 540 may be distributed at at least two positions of the first magnetic attraction position 501 in a horizontal circumferential direction, or may be distributed at a single side of the first magnetic attraction position 501, as long as it is ensured that the magnetic field provided by the first magnetic assembly 540 to the first magnetic attraction position 501 can form at least two spaced magnetic bead groups 40 on the inner side wall of the reaction vessel 20 located at the first magnetic attraction position 501 by attraction.

As an implementation, the first liquid aspiration position 502 is formed in between second magnetic assembly 550 or at a side of the second magnetic assembly 550, that is, the second magnetic assembly 550 may be distributed at at least two positions of the first liquid aspiration position 502 in a horizontal circumferential direction, or may be distributed at a single side of the first liquid aspiration position 502, as long as it is ensured that the magnetic field provided by the second magnetic assembly 550 to the first liquid aspiration position 502 can form at least one magnetic bead group 40 on the inner side wall of the reaction vessel 20 located at the first liquid aspiration position 502 by attraction, and the number of magnetic bead groups 40 formed by attraction at the first liquid aspiration position is less than the number of magnetic bead groups 40 formed by attraction at the first magnetic attraction position 501.

As an implementation, the first magnetic assembly 540 includes (or, is composed of) a pair of first magnetic devices 541 arranged at an interval, a first magnetic attraction position 501 is formed between the pair of first magnetic devices 541 arranged at an interval or at the same side of the pair of first magnetic devices 541 arranged at an interval, and the first magnetic assembly 540 is configured to perform a first magnetic attraction action on the first reaction solution in a reaction vessel 20 located at the first magnetic attraction position 501, so as to form two spaced magnetic bead groups 40 on an inner side wall of the reaction vessel 20 located at the first magnetic attraction position 501 by attraction. The second magnetic assembly 550 is a single second magnetic device 551, a first liquid aspiration position 502 is formed at a side of the single second magnetic device 551, the second magnetic assembly 550 is configured to perform a second magnetic attraction action on the first reaction solution in a reaction vessel 20 located at the first liquid aspiration position 502, to form a single magnetic bead group 40 on an inner side wall of the reaction vessel 20 located at the first liquid aspiration position 502 by attraction. The pair of first magnetic devices 541 arranged at an interval at the first magnetic attraction position 501 may attract, from different directions, magnetic beads and substances combined with the magnetic beads, so that distances moved by the magnetic beads and substances combined with the magnetic beads when they are attracted may be shortened, and the magnetic beads and substances combined with the magnetic beads may be quickly attracted to the inner side wall of the reaction vessel 20 nearby, which is beneficial to ensure the attraction speed at the first magnetic attraction position 501. Furthermore, a single second magnetic device 551 located at the first liquid aspiration position 502 may aggregate all magnetic beads and substances combined with the magnetic beads in the reaction vessel 20 located at the first liquid aspiration position 502, into a magnetic bead group 40 by attraction, and therefore it is beneficial to improve attraction stability of the magnetic beads and substances combined with the magnetic beads during liquid aspiration. In this implementation, the first magnetic assembly 540 forms two spaced magnetic bead groups 40 on the inner side wall of the reaction vessel 20 located at the first magnetic attraction position 501 by attraction, the second magnetic assembly 550 forms a single magnetic bead groups 40 on the inner side wall of the reaction vessel 20 located at the first liquid aspiration position 502 by attraction, which meets a design requirement that the number of magnetic bead groups 40 formed by the first magnetic assembly 540 on the inner side wall of the reaction vessel 20 located at the first magnetic attraction position 501 by attraction is greater than the number of magnetic bead groups 40 formed by the second magnetic assembly 550 on the inner side wall of the reaction vessel 20 located at the first liquid aspiration position 502 by attraction. Of course, in a specific application, the number of magnetic bead groups 40 formed by the first magnetic assembly 540 on the inner side wall of the reaction vessel 20 located at the first magnetic attraction position 501 by attraction and the number of magnetic bead groups 40 formed by the second magnetic assembly 550 on the inner side wall of the reaction vessel 20 located at the first liquid aspiration position 502 by attraction are not limited thereto. For example, as an alternative implementation, through adjusted design of the first magnetic assembly 540 and the second magnetic assembly 550, the first magnetic assembly 540 may form three or more magnetic bead groups 40 at intervals on the inner side wall of the reaction vessel 20 located at the first magnetic attraction position 501 by attraction, the second magnetic assembly 550 may form a single magnetic bead group 40 or two magnetic bead groups 40 arranged at an interval on the inner side wall of the reaction vessel 20 located at the first liquid aspiration position 502 by attraction.

As an implementation, the two first magnetic devices 541 are arranged at opposite sides of the first magnetic attraction position 501 at an interval in a horizontal direction, that is, the pair of first magnetic devices 541 arranged at an interval are arranged at opposite sides of the first magnetic attraction position 501 at an interval in the horizontal direction. In this implementation, first magnetic assemblies 540 are distributed at opposite sides of the first magnetic attraction position 501, that is, one of the first magnetic devices 541 is arranged at one side of the first magnetic attraction position 501, and the other one of the first magnetic devices 541 is arranged at the other side of the first magnetic attraction position 501. In this way, the pair of first magnetic devices 541 arranged at an interval at the first magnetic attraction position 501 may attract magnetic beads and substances combined with the magnetic beads from opposite sides of the reaction vessel 20, so that distances moved by the magnetic beads and substances combined with the magnetic beads when they are attracted may be shortened, and the magnetic beads and substances combined with the magnetic beads may be quickly attracted to the inner side wall of the reaction vessel 20 nearby, which is beneficial to ensure the attraction speed at the first magnetic attraction position 501. The second magnetic assembly 550 is distributed at a single side of the first liquid aspiration position 502, which is beneficial to ensure attraction stability of the magnetic beads and the target substance combined with the magnetic beads in the reaction vessel 20 located at the first liquid aspiration position 502. In this implementation, the magnetic field configured at the first magnetic attraction position 501 is as follows. Magnets are arranged at opposite sides of the first magnetic attraction position 501, to shorten flight distances of the magnetic beads and enable the magnetic beads to be quickly attracted to the inner wall of the reaction vessel 20. The magnetic field configured at the first liquid aspiration position 502 is as follows. A magnet is arranged at a single side of the first liquid aspiration position 502, to aggregate all magnetic beads into one point, so that stability of the magnetic beads during liquid aspiration can be improved, and a combination of a magnetic field design function of quick attraction at the first magnetic attraction position 501 and a magnetic field design function of stable attraction at the first liquid aspiration position 502 is achieved. By arranging a magnetic field capable of quickly attracting the magnetic beads at the first magnetic attraction position 501 where only magnetic attraction is performed and liquid aspiration is not performed, and arranging a magnetic field capable of stabilizing the magnetic beads at the first liquid aspiration position 502, loss of magnetic beads at each of the two steps in the magnetic separation process can be reduced, so as to minimize a total loss of magnetic particles.

As an implementation, the pair of first magnetic devices 541 arranged at an interval are arranged at opposite sides of the first magnetic attraction position 501 at an interval in a diametrical direction of the same reaction vessel 20. Two magnetic bead groups 40 formed by the first magnetic assembly 540 on the inner side wall of the reaction vessel 20 located at the first magnetic attraction position 501 though attraction are distributed at opposite sides of the reaction vessel 20 at an interval in the diametrical direction of the reaction vessel 20.

As an implementation, the transfer assembly is configured to drive the holding assembly, to move the reaction vessel 20 loaded with the first reaction solution after incubation to the first magnetic attraction position 501 and the first liquid aspiration position 502 in sequence; two first magnetic devices 541 are arranged at opposite sides of a movement trajectory of movement of the reaction vessel 20 driven by the transfer assembly respectively (the movement trajectory is described below, and refers to the movement trajectory of movement of the reaction vessel 20 driven by the transfer assembly, unless specifically stated), and the second magnetic device 551 is arranged at a side of the movement trajectory. In this implementation, a transfer mechanism enables the holding assembly to move the reaction vessel 20 to the first magnetic attraction position 501 and the first liquid aspiration position 502 respectively by way of driving the holding assembly to move, so that it is unnecessary for the transfer assembly to perform actions of clamping and releasing the reaction vessel 20, and therefore it is beneficial to simplify structure of the transfer assembly and improve efficiency of transfer the reaction vessel 20 by the transfer assembly. Of course, in a specific application, as an alternative implementation, the transfer assembly may be designed to move the reaction vessel 20 to the first magnetic attraction position 501 and the first liquid aspiration position 502 respectively by way of clamping the reaction vessel 20 to move. For example, the transfer assembly includes a gripper configured to clamp the reaction vessel 20 and a power component configured to drive the gripper to move.

As an implementation, the holding assembly is a magnetic separation disk 510, that is, the holding assembly is of a disk shape. The holding assembly is formed with multiple accommodation grooves arranged in the horizontal circumferential direction, each accommodation groove is configured to accommodate a reaction vessel 20, and the transfer assembly is configured to drive the holding assembly to horizontally rotate the reaction vessel 20 around a central axis of the holding assembly, so that the reaction vessel 20 accommodated in the accommodation groove is transferd to the first magnetic attraction position 501 and the first liquid aspiration position 502 in sequence. The trajectory of movement of the reaction vessel 20 driven by the transfer assembly is a circular trajectory, and the first magnetic attraction position 501 and the first liquid aspiration position 502 are distributed along the circular trajectory. Specifically, when the transfer assembly drives the holding assembly to rotate, the reaction vessel 20 on the holding assembly may pass through the first magnetic attraction position 501 and the first liquid aspiration position 502 in sequence. Of course, in a specific application, the shape of the holding assembly is not limited thereto, and the trajectory of movement of the reaction vessel 20 driven by the transfer assembly is not limited thereto. For example, as an alternative implementation, the trajectory of movement of the reaction vessel 20 driven by the transfer assembly is a linear trajectory, and the first magnetic attraction position 501 and the first liquid aspiration position 502 are distributed along the linear trajectory. Or, as another alternative implementation, the trajectory of movement of the reaction vessel 20 driven by the transfer assembly is a meander line trajectory, and the first magnetic attraction position 501 and the first liquid aspiration position 502 are distributed along the meander line trajectory.

As an implementation, the first magnetic assembly 540 and the second magnetic assembly 550 are arranged in the magnetic separation disk 510, to magnetically attract the first reaction solution in the magnetic separation disk 510.

As an implementation, the magnetic separation device 500 includes a magnetic separation disk 510 and a lid 520. The magnetic separation disk 510 is configured to accommodate multiple reaction vessels 520. The lid 520 covers the magnetic separation disk 510; the liquid path device 420 further includes a main branch 422, a confluence component 421 and a liquid injection branch 423. The liquid injection branch 423 includes a liquid injection guide channel 4232 formed in the confluence component 421 and a first liquid injection component 4231 at least partially arranged outside the confluence component 421 to dispense the cleaning liquid. The main branch 422 is communicated with the first liquid injection component 4231 through the confluence component 421. The confluence component 421 is mounted on the lid 520. In this implementation, the confluence component 421 is directly mounted on the lid 520 of the magnetic separation device 500, and the confluence component 421, the first liquid injection component 4231 and pipeline are mounted without providing an additional bracket, thereby simplifying structure of the sample analyzer 10 to a great extent.

As an implementation, the first magnetic device 541 includes (or, is composed of) at least two first magnets 5411. In this implementation, the first magnetic device 541 includes (or, is composed of) at least two first magnets 5411, each first magnet 5411 may form a magnetic field. It may be beneficial to enhance magnetic attraction force by superimposing magnetic fields of at least two first magnets 5411, therefore it is beneficial to improve magnetic attraction effect and efficiency. Of course, in a specific application, as an alternative implementation, the first magnetic device 541 may include (or, be composed of) only one first magnet 5411.

As an implementation, the first magnetic device 541 includes (or, is composed of) two first magnets 5411, each having at least one end abutted against at least one end of another one of the magnets. There may be the following cases for the composition of the first magnetic device 541 including (or, composed of) the two first magnets 5411: the first magnetic device 541 includes (or, is composed of) two first magnets 5411 stacked in the vertical direction, the first magnetic device 541 includes (or, is composed of) two first magnets 5411 abutted against each other in the horizontal circumferential direction, or the first magnetic device 541 includes (or, is composed of) two first magnets 5411 abutted against each other in an oblique direction. In this implementation, the number of the first magnets 5411 in the first magnetic device 541 is 2, which is also beneficial for the first magnetic device 541 not to generate a problem of increasing cost due to an excessive number of the first magnets 5411, on the premise of enhancing magnetic attraction force of the first magnetic device 541. Of course, in a specific application, as an alternative implementation, the first magnetic device 541 may include (or, be composed of) three or more first magnets 5411, each having at least one end abutted against at least one end of another one of the magnets.

As an implementation, the second magnetic device 551 includes (or, is composed of) at least two second magnets 5511. In this implementation, the second magnetic device 551 includes (or, is composed of) at least two second magnets 5511, each second magnet 5511 may form a magnetic field. It may be beneficial to enhance the magnetic attraction force by superimposing magnetic fields formed by at least two second magnets 5511, and therefore it is beneficial to improve magnetic attraction effect and efficiency. Of course, in a specific application, as an alternative implementation, the second magnetic device 551 may include (or, be composed of) only one second magnet 5511.

As an implementation, the first magnetic device 541 includes (or, is composed of) two first magnets 5411 stacked in the vertical direction, and the second magnetic device 551 includes (or, is composed of) two second magnets 5511 stacked in the vertical direction. With this arrangement solution, it is also beneficial to reduce horizontal space occupied by the first magnetic device 541 and the second magnetic device 551, on the premise of enhancing the magnetic attraction force of the first magnetic device 541 and the second magnetic device 551.

As an implementation, the magnetic separation device 500 further includes at least one third magnetic assembly 560. The third magnetic assembly 560 includes (or, is composed of) a single third magnetic device 561, and a second magnetic attraction position 503 is formed at a side of the single third magnetic device 561. The third magnetic assembly 560 is configured to perform a third magnetic attraction action on the first reaction solution in a reaction vessel 20 located at the second magnetic attraction position 503, to form a single magnetic bead group 40 on an inner side wall of the reaction vessel 20 located at the second magnetic attraction position 503 by attraction. The operation of transfer the reaction vessel 20 after completing the first magnetic attraction action to the first liquid aspiration position 502 by the transfer assembly, includes that, the transfer assembly transfers the reaction vessel 20 after completing the first magnetic attraction action to the second magnetic attraction position 503, and then transfers the reaction vessel 20 after completing the third magnetic attraction action to the first liquid aspiration position 502. In this implementation, after the first magnetic assembly 540 quickly forms two spaced magnetic bead groups 40 at the first magnetic attraction position 501 by attraction, the third magnetic assembly 560 aggregates the two spaced magnetic beads 40 at the second magnetic attraction position 503 to form a magnetic bead group 40 by attraction, so that after the magnetic bead group 40 is attracted stably, magnetic attraction and liquid aspiration is performed at the first liquid aspiration position 502, therefore it may further ensure attraction stability of the magnetic bead group 40 during liquid aspiration.

As an implementation, configuration of the third magnetic device 561 is the same as that of the second magnetic device 551, which is not described in detail here.

As an implementation, the first antigen-antibody binder contained in the first reaction solution is generated by an antigen-antibody binding reaction among the sample, a magnetic bead reagent and a marker reagent, that is, the first antigen-antibody binder is generated by binding according to a sandwich method. Of course, in a specific application, as an alternative implementation, the first antigen-antibody binder in the reaction solution may be generated by binding according to a competitive method, in which the first antigen-antibody binder contained in the first reaction solution is generated by an antigen-antibody binding reaction between the sample and the magnetic bead reagent, and the first reaction solution contains a second antigen-antibody binder generated by an antigen-antibody binding reaction between the marker reagent and the magnetic bead reagent, in addition to the first antigen-antibody binder generated by an antigen-antibody binding reaction between the sample and the magnetic bead reagent. Specific detection principles of the sandwich method and the competitive method are described below.

As an implementation, the reagent dispensing device 200 is further configured to dispense a magnetic bead reagent and a marker reagent to the reaction vessel 20 before the magnetic separation cleaning operation, the magnetic bead reagent is provided with an antibody or an antigen, the marker reagent is an antibody reagent provided with a luminescent marker or an antigen reagent provided with a luminescent marker; the first antigen-antibody binder contained in the first reaction solution is generated by a binding reaction among at least two of the following: the target antigen or the target antibody in the sample, the antibody or antigen in the magnetic bead reagent, and the antibody or antigen in the marker reagent; that is, the first antigen-antibody binder is generated by an antigen-antibody binding reaction among at least two of the following: the sample, the magnetic bead reagent, and the marker reagent. The operation of analyzing by controller 102 to obtain the detection result of the sample includes: the controller 102 performs analyzing, to obtain a content of the target antigen in the sample or a content of the target antibody in the sample.

As an implementation, the magnetic bead reagent and the marker reagent may be dispensed into the reaction vessel 20 simultaneously; or, the magnetic bead reagent may be dispensed into the reaction vessel 20, and then the marker reagent may be dispensed into the reaction vessel 20; or, the marker reagent may be dispensed into the reaction vessel 20, and then the magnetic bead reagent may be dispensed into the reaction vessel 20.

As an implementation, the sample analyzer 10 further includes an incubation device 900. The incubation device 900 is configured to incubate the reaction solution in the reaction vessel 20. The reagent dispensing device 200 is configured to dispense the magnetic bead reagent and the marker reagent to the reaction vessel 20 in the incubation device 900 before the magnetic separation cleaning operation. The incubation device 900 is at least configured to incubate the sample, the magnetic bead reagent and the marker reagent, to form the first reaction solution.

As an implementation, the sample analyzer 10 further includes a substrate dispensing device 101. The substrate dispensing device 101 is configured to dispense, after the magnetic separation cleaning operation, a luminescent substrate reagent to the second reaction solution, to prepare a third reaction solution, that is, the substrate dispensing device 101 is configured to dispense a luminescent substrate reagent to the second reaction solution prepared by the magnetic separation cleaning operation, to prepare a third reaction solution. The luminescent substrate reagent is configured to generate light with a predetermined wavelength under catalysis of the luminescent marker in the first antigen-antibody binder or the second antigen-antibody binder (in the sandwich method, the luminescent marker is located in the first antigen-antibody binder; in the competitive method, the luminescent marker is located in the second antigen-antibody binder). The operation of detecting the third reaction solution by the sample detection device 300 includes: detecting an optical signal in the third reaction solution. The operation of analyzing information of the optical signal to obtain the detection result of the sample by the controller 102, includes that, the controller 102 analyzes information of the optical signal detected by the sample detection device 300, to obtain a content of the target antigen or the target antibody in the sample. In this implementation, the substrate dispensing device 101 is separately provided to dispense the luminescent substrate reagent to the second reaction solution, that is, the substrate dispensing device 101 and the reagent dispensing device 200 are independent of each other. In this way, dispensing of the magnetic bead reagent and the marker reagent for a sample detection item and dispensing of the luminescent substrate reagent for another sample detection item may be performed at the same time, which is further beneficial to improve efficiency of batch detection of samples. Of course, in a specific application, as an alternative implementation, the luminescent substrate reagent may be dispensed by the reagent dispensing device 200 without providing the substrate dispensing device 101 independent of the reagent dispensing device 200, that is, the reagent dispensing device 200 is further configured to dispense, after magnetic separation, the luminescent substrate reagent to the second reaction solution, to prepare the third reaction solution.

As an implementation, the sample detection device 300 is configured to perform sample detection on the third reaction solution in the incubation device 900. In this implementation, the incubation device 900 is configured to carry the reaction vessel 20 to perform sample detection, in addition to carrying the reaction vessel 20 to incubate the sample and the reagent in the reaction vessel 20 to obtain the first reaction solution.

As an implementation, the sample detection device 300 is an optical detection device, and the optical detection device is arranged next to the incubation device 900, to detect the optical signal in the third reaction solution in the reaction vessel 20 in the incubation device 900.

As an implementation, the detection item performed by the sample analyzer 10 includes at least an immunoassay luminescence detection item. An aim of immunoassay luminescence detection is to detect a content of a specific antigen or antibody in the sample. Various kinds of impurities are present in the sample, some impurities such as endogenous enzyme contained in a blood sample itself may affect a final detection system (i.e., a final to-be-detected reaction solution) and thus affect accuracy of the detection result. Therefore, the process of immunoassay luminescence detection is mainly intended to purify the antigen or the target antibody, and finally obtain a detection level of the target antigen or the target antibody in the sample through luminescence detection of markers. In this implementation, in order to detect the level of the target antigen or the target antibody in the sample, concentration of the target antigen or the target antibody is converted into a luminescent physical quantity. Since the antigen or the antibody itself does not have capability of luminescence, conversion of luminescence is perform by the antibody provided with a luminescent marker or the antigen provided with a luminescent marker.

As an implementation, the first antigen-antibody binder contained in the first reaction solution is formed by binding the antigen to the antibody according to the sandwich method. For example, if the content of the target antigen in the sample is to be detected, the magnetic bead reagent dispensed by the reagent dispensing device 200 is provided with an antibody, the marker reagent is provided with a luminescent marker, and the target antigen in the sample is sandwiched between the antibody of the magnetic bead reagent and the antibody of the marker reagent. In this solution, a cell surface of the sample is provided with an antigen, a surface of the magnetic bead is provided with an antibody, and the marker reagent is provided with an antibody. The target antigen in the sample may be specifically bound to the magnetic bead provided with a specific antibody and the antibody provided with a luminescent marker, to form the first antigen-antibody binder of magnetic bead-target antigen-antibody of the luminescent marker. If the content of the target antibody in the sample is to be detected, the magnetic bead reagent dispensed by the reagent dispensing device 200 is provided with an antigen, the marker reagent is an antibody reagent provided with a luminescent marker or an antigen reagent provided with a luminescent marker, and the target antibody in the sample is sandwiched between the antigen of the magnetic bead reagent and the antibody of the marker reagent or between the antigen of the magnetic bead reagent and the antigen of the marker reagent.

As an implementation, the process of immunoassay luminescence detection according to the sandwich method includes: in a process of initially mixing the sample and the reagent, the target antigen is bound to the magnetic bead provided with a specific antibody, and then the target antigen is specifically bound to the antibody provided with a luminescent marker in the reagent, at this time, a system of magnetic bead-target antigen-antibody of the luminescent marker (i.e., the above first antigen-antibody binder) is generated. After the system of magnetic bead-target antigen-antibody of the luminescent marker is generated, it enters a step of the magnetic separation cleaning operation. Before the step of the magnetic separation cleaning operation, the reaction system contains impurities, and magnetic separation cleaning may clean off the impurities by changing solutions and re-dispersing, while retaining the to-be-detected target antigen in the reaction vessel 20, thereby achieving purification. Finally, the purified reaction solution is mixed with the luminescent substrate reagent, the luminescent substrate reagent generates light with a certain wavelength and a certain frequency under catalysis of the luminescent marker. The light is collected and counted by the sample detection device 300 (such as a photometer, etc.), and the controller 102 calculates a concentration value of the corresponding target antigen according to data fed back by the sample detection device 300 and thereby obtains a target antigen level of the corresponding sample, to help clinical judgment.

Of course, in a specific application, the antigen-antibody binder contained in the first reaction solution is not limited to being formed by binding the antigen to the antibody according to the sandwich method. In an alternative implementation, the antigen-antibody binder contained in the first reaction solution may be formed by binding the antigen to the antibody according to the competitive method. In this alternative solution, the antigen-antibody binder contained in the first reaction solution includes a first antigen-antibody binder formed by binding the target antigen in the sample to the antibody on the surface of the magnetic bead in the magnetic bead reagent, and a second antigen-antibody binder formed by binding the antigen in the marker reagent to the antibody on the surface of the magnetic bead in the magnetic bead reagent. Since there is a limited number of antibodies on the magnetic bead, the target antigen in the sample and the antigen in the marker reagent form a competitive relationship, that is, a luminescence value is inversely proportional to concentration of a target substance in the sample. Therefore, the concentration of the target substance in the sample may be calculated backward through the measured luminescence value.

As an implementation, the sample analyzer 10 further includes a transfer device 104. The transfer device 104 is configured to transfer the reaction vessel 20. The transfer device 104 is configured to perform at least one of transfer operations as follows: transferring the reaction vessel 20 after completing sample loading to the incubation device 900; transferring the reaction vessel 20 loaded with the first reaction solution to the magnetic separation device 500; transferring the reaction vessel 20 after completing magnetic separation to the incubation device 900 for detection; or transferring the reaction vessel 20 after detection to a cup throwing position, to discard and recover the reaction vessel.

As an implementation, the sample analyzer 10 further includes a reaction vessel providing device 103 and a reaction vessel recovery device 105. The reaction vessel providing device 103 is configured to provide the reaction vessel 20, and the reaction vessel recovery device 105 is configured to recover the reaction vessel 20. The sample analyzer 10 further has a recovery position, and the reaction vessel recovery device 105 is located below the recovery position. The transfer device 104 is at least configured to transfer the reaction vessel 20 from the incubation device 900 to the recovery position, to release and recover the reaction vessel. In this implementation, the reaction vessel 20 is a disposable vessel, that is, a reaction vessel 20 is recovered and processed after completing a detection item. Of course, in a specific application, the reaction vessel 20 may be a vessel that can be recycled, that is, after the reaction vessel 20 completes a detection item, it may be cleaned in the sample analyzer 10 and then reused to perform other detection items.

As an implementation, the sample analyzer 10 is a non-constant speed immunoassay analyzer. With the above solutions, a problem of deviation of measured values due to an unstable temperature of the cleaning liquid in initial few sample detections of the non-constant speed immunoassay analyzer may be solved effectively, while influence of sensitivity and precision of detection items due to a long non-backflow end and inaccurate temperature control (because of lack of temperature control) may be reduced. Specifically, with regard to detection items which are sensitive to temperature and require backflow of the cleaning liquid, usage of means of bidirectional circulation temperature control and reducing non-backflow sections in this embodiment can improve accuracy of the temperature of the cleaning liquid, thereby reducing influence of variation of the temperature of the cleaning liquid on the detection result of the sample, improving inclusiveness of the non-constant speed immunoassay analyzer to development of reagents and improving signal-to-noise ratios of temperature-sensitive reagents, and solving the problem of poor precision of liquid injection due to volatilization of the cleaning liquid at high temperature. Of course, in a specific application, the sample analyzer 10 according to this embodiment is not limited to the immunoassay analyzer, for example, is also applicable to other analyzers requiring bidirectional temperature control of the cleaning liquid.

In the non-constant speed immunoassay analyzer, backflow is a mainstream choice in the temperature control solution of the cleaning liquid, and backflow is intended to enable the cleaning liquid to repeatedly circulate in the heat exchanger 410, so as to ensure stability of the temperature of the cleaning liquid leaving the confluence component 421 each time. However, if a liquid injection part of the cleaning liquid leaving the backflow component and leading to the liquid injection port of the first liquid injection component 4231 (a liquid injection terminal) is not subjected to a thermal insulation measure or only subjected to thermal insulation by thermal insulation materials such as thermal insulation cottons, it is easy to cause an inaccurate liquid injection temperature of the cleaning liquid in continuous detections and a phenomenon that the temperature of the cleaning liquid is completely out of control in the initial few detections of discontinuous detections. With regard to this problem, this embodiment starts from a length of the section of non-backflow zone, reduces the non-backflow zone as much as possible, and combines the backflow valve (i.e., the switching device 424) with the confluence block (i.e., the confluence component 421), i.e., the backflow valve is combined with the confluence block, and the backflow valve is directly mounted on the confluence block, thereby reducing an amount of non-backflow liquid to a great extent, and further ensuring accuracy and reliability of the temperature of the cleaning liquid supplied for each detection.

In order to improve accuracy of temperature control of liquid injection and accuracy of detection, the sample analyzer 10 according to this embodiment directly connects the terminal of the backflow structure to the terminal of the liquid injection structure, thereby reducing the length of the non-backflow section (it is not easy to perform temperature control on this section) and reducing a length of heat loss in the whole liquid path design. Furthermore, in this embodiment, accuracy of temperature control of liquid injection is improved, and influence of inaccurate temperature on the luminescence value is reduced, by optimizing design of the temperature control device.

As an implementation, the operation process of the cleaning liquid dispensing device 400 includes that, the cleaning liquid flows to the heat exchanger 410, and the heat exchanger 410 cools or heats the cleaning liquid flowing through it. Then, the cooled or heated cleaning liquid flows to the confluence component 421 and then to the switching device 424. If the cleaning liquid dispensing device 400 is in an operation state of requiring liquid injection, the liquid injection port of the switching device 424 is open, the backflow port c is closed, the cleaning liquid leaves the switching device 424, enters the liquid injection branch 423, and finally is injected into the first reaction solution of the magnetic separation device 500 through the first liquid injection component 4231. If the cleaning liquid dispensing device 400 is in an operation state of standby, the backflow port c of the switching device 424 is open, the liquid injection port is closed, the cleaning liquid flows back to the heat exchanger 410 from the backflow port c of the switching device 424, and continues to circulate and perform heat exchange for keeping the temperature.

### Second embodiment:

The sample analyzer 10 according to this embodiment differs from the first embodiment mainly in that application scenarios of the cleaning liquid supplied by the cleaning liquid dispensing device 400 may be different those in the first embodiment. For example, in this embodiment, the cleaning liquid supplied by the cleaning liquid dispensing device 400 is not limited to being applied to application scenarios in which the magnetic separation device 500 is included.

Specifically, the sample analyzer 10 according to this embodiment includes a sample dispensing device 100, a reagent dispensing device 200, a sample detection device 300, a cleaning liquid dispensing device 400 and a controller 102. The sample dispensing device 100 is configured to aspirate a sample from a sample vessel and dispense at least a part of the aspirated sample to a reaction vessel 20. The reagent dispensing device 200 is configured to aspirate a reagent from a reagent vessel and dispense the aspirated reagent to the reaction vessel 20. The sample detection device 300 is configured to detect a reaction solution made of at least the sample and the reagent. The controller 102 is configured to control operations of the sample dispensing device 100, the reagent dispensing device 200, the sample detection device 300 and the cleaning liquid dispensing device 400, and configured to analyze detection information fed back by the sample detection device 300, so as to obtain a detection result of the sample. The cleaning liquid dispensing device 400 is provided with a function of heating the cleaning liquid circularly and a function of cooling the cleaning liquid circularly, and the cleaning liquid dispensing device 400 is configured to supply the heated or cooled cleaning liquid that reaches a temperature within a target temperature range to at least one of the reaction vessel 20, the sample dispensing device 100 or the reagent dispensing device 200. The cleaning liquid dispensing device 400 according to this embodiment may heat the cleaning liquid circularly or may cool the cleaning liquid circularly, to supply the cleaning liquid within different temperature ranges, for example, supply the cleaning liquid with normal atmospheric temperature substantially consistent with ambient temperature, or supply the cleaning liquid with high temperature higher than ambient temperature, or supply the cleaning liquid with low temperature lower than ambient temperature, to meet requirements of the cleaning liquid with different temperatures in different application scenarios.

As an implementation, the cleaning liquid dispensing device 400 includes a heat exchanger 410, a liquid path device 420 and a power device 430. The heat exchanger 410 is configured to perform heating and cooling respectively under control of the controller 102. The liquid path device 420 is configured to provide a flow path for a cleaning liquid, and at least a part of the liquid path device 420 is in contact with the heat exchanger 410, so as to allow the cleaning liquid in the liquid path device 420 to be heated or cooled by the heat exchanger 410 to reach a temperature within a target temperature range. The power device 430 is connected to the liquid path device 420 to drive, under control of the controller 102, the liquid path device 420 to aspirate a cleaning liquid from a cleaning liquid container 30, and drive, under control of the controller 102, the liquid path device 420 to dispense the cleaning liquid that reaches a temperature within the target temperature range through heating or cooling by the heat exchanger 410, to the first reaction solution in the magnetic separation device 500. The liquid path device 420 includes a main branch 422, a backflow branch 425, a liquid injection branch 423 and a switching device 424, one end of the main branch 422 is configured to aspirate the cleaning liquid from the cleaning liquid container 30, the other end of the main branch 422 is connected to the switching device 424 that switches between the backflow branch 425 and the liquid injection branch 423, and at least a part of the main branch 422 forms a heat exchange tube section 4201 in contact with the heat exchanger 410, so as to allow the cleaning liquid in the heat exchange tube section 4201 to be heated or cooled by the heat exchanger 410. The power device 430 is connected to the main branch 422. The liquid injection branch 423 is configured to dispense the cleaning liquid. The backflow branch 425 forms a circulation loop with the main branch 422 and the cleaning liquid container 30, or the backflow branch 425 forms a circulation loop with the main branch 422, so that the cleaning liquid outputted from the main branch 422 is guided by the backflow branch 425 to circulate through the heat exchanger 410, so as to exchange heat.

As an implementation, a liquid path formed by the liquid injection branch 423 has a length less than or equal to 200 mm.

As an implementation, the liquid path formed by the liquid injection branch 423 has a length less than or equal to 20 mm.

As an implementation, a volume of the liquid injection branch 423 is less than or equal to 10% of a liquid amount of the cleaning liquid that is dispensed by the liquid injection branch 423 at one time.

As an implementation, a volume of the liquid injection branch 423 is less than or equal to 5% of the liquid amount of the cleaning liquid that is dispensed by the liquid injection branch 423 at one time.

As an implementation, the switching device 424 is formed with a liquid inlet *a*, a backflow port c and a liquid outlet *b.* The liquid inlet *a* is configured to enable the cleaning liquid after heat exchange through the heat exchanger 410 to flow into the switching device 424. The backflow port *c* is configured to enable the cleaning liquid flowing into the switching device 424 to flow back to the main branch 422 through the backflow branch 425, so as to exchange heat with the heat exchanger 410. The liquid outlet *b* is configured to output the cleaning liquid to the liquid injection branch 423. The liquid path device 420 further includes a confluence component 421. The switching device 424 is mounted on the confluence component 421, and the liquid outlet *b* is abutted against the confluence component 421. The liquid injection branch includes a liquid injection guide channel 4232 formed in the confluence component 421 and a first liquid injection component 4231 at least partially arranged outside the confluence component 421 to dispense the cleaning liquid, and each of two ends of the liquid injection guide channel 4232 is communicated with a respective one of the liquid outlet *b* and the first liquid injection component 4231.

As an implementation, the controller 102 is further configured to control the target temperature range for the magnetic separation cleaning operation for the at least one sample detection item to be within 10°C to 35°C, that is, the temperature in the target temperature range for each of the at least one sample detection item is equal to or greater than 10°C and less than or equal to 35°C.

As an implementation, the controller 102 is further configured to control the target temperature range for the magnetic separation cleaning operation for the at least one sample detection item to be within 15°C to 30°C, that is, the temperature in the target temperature range for each of the at least one sample detection item is equal to or greater than 15°C and less than or equal to 30°C.

As an implementation, the controller 102 is further configured to control the target temperature range for the magnetic separation cleaning operation for the at least one sample detection item to be within 24°C to 30°C, that is, the temperature in the target temperature range for each of the at least one sample detection item is equal to or greater than 24°C and less than or equal to 30°C.

As an implementation, the controller 102 is further configured to control the target temperature range for the magnetic separation cleaning operation for the at least one sample detection item to be within 25°C to 29°C, that is, each temperature in the target temperature range for the at least one sample detection item is within a range of 25°C to 29°C.

Besides the above descriptions, other parts of the sample analyzer 10 according to this embodiment may refer to the first embodiment, which are not described in detail here.

### Third embodiment:

The sample analyzer 10 according to this embodiment differs from the first embodiment mainly in that the cleaning liquid dispensing device 400 in this embodiment is not necessarily provided with both heating and cooling functions, and instead, it only needs a temperature of the cleaning liquid output to be within a target temperature range.

Specifically, the sample analyzer 10 according to this embodiment includes a sample dispensing device 100, a reagent dispensing device 200, a sample detection device 300, a magnetic separation device 500, a cleaning liquid dispensing device 400 and a controller 102. The sample dispensing device 100 is configured to aspirate a sample from a sample vessel and dispense at least a part of the aspirated sample to a reaction vessel 20. The reagent dispensing device 200 is configured to aspirate a reagent from a reagent vessel and dispense the aspirated reagent to the reaction vessel 20. The reaction vessel 20 is at least configured to provide a reaction place for the sample and the reagent, so that a first reaction solution containing at least a first antigen-antibody binder is generated by an antigen-antibody binding reaction between a target antigen in the sample and an antibody in the reagent or between a target antibody in the sample and an antigen in the reagent. The cleaning liquid dispensing device 400 is configured to provide a cleaning liquid within a target temperature range. The magnetic separation device 500 is configured to perform a magnetic separation cleaning operation on the first reaction solution through the cleaning liquid that is within the target temperature range provided by the cleaning liquid dispensing device 400, to prepare a second reaction solution. A substrate dispensing device 101 is configured to dispense a luminescent substrate reagent to the second reaction solution prepared by the magnetic separation cleaning operation, to prepare a third reaction solution. The sample detection device 300 is configured to perform an optical signal detection on the third reaction solution. The controller 102 is configured to control operations of the sample dispensing device 100, the reagent dispensing device 200, the sample detection device 300, the magnetic separation device 500, the substrate dispensing device 101 and the cleaning liquid dispensing device 400, and configured to analyze information of the optical signal detected by the sample detection device 300, so as to obtain a content of the target antigen in the sample or a content of the target antibody in the sample. The controller 102 is further configured to control a target temperature range for a magnetic separation cleaning operation for at least one sample detection item to be within 10°C to 35°C. Controlling the temperature of the cleaning liquid to be within a range of 10°C to 35°C may reduce the phenomenon of dissociation of the antigen-antibody binder, which effectively ensures accuracy of the sample detection result and improves clinical performance of temperature-sensitive items. Furthermore, controlling the temperature of the cleaning liquid outputted from the cleaning liquid dispensing device 400 to be within a range of 10°C to 35°C, may meet application requirements of the cleaning liquid tending to normal atmospheric temperature and low temperature, thereby improving compatibility of the sample analyzer 10 on development of low temperature reagents, for example, reducing consideration of temperature during development of magnetic bead reagents and marker reagents, and solving the problem of poor precision of liquid injection due to volatilization of the cleaning liquid at high temperature.

As an implementation, the controller 102 is further configured to control the target temperature range for the magnetic separation cleaning operation for the at least one sample detection item to be within 15°C to 30°C, that is, the temperature in the target temperature range for each of the at least one sample detection item is equal to or greater than 15°C and less than or equal to 30°C.

As an implementation, the controller 102 is further configured to control the target temperature range for the magnetic separation cleaning operation for the at least one sample detection item to be within 24°C to 30°C, that is, the temperature in the target temperature range for each of the at least one sample detection item is equal to or greater than 24°C and less than or equal to 30°C.

As an implementation, the controller 102 is further configured to control the target temperature range for the magnetic separation cleaning operation for the at least one sample detection item to be within 25°C to 29°C, that is, the temperature in the target temperature range for each of the at least one sample detection item is within a range of 27°C±2°C.

As an implementation, the cleaning liquid dispensing device 400 is provided with at least a function of cooling the cleaning liquid circularly to make the cleaning liquid reach a temperature within the target temperature range. A solution similar to that in the first embodiment may be used for the solution of the cleaning liquid dispensing device 400 cooling the cleaning liquid circularly, except that the heat exchanger 410 in this embodiment does not necessarily require a heating function.

As an implementation, the reagent dispensing device 200 is further configured to dispense a magnetic bead reagent and a marker reagent to the reaction vessel 20 before the magnetic separation cleaning operation. The magnetic bead reagent is provided with an antibody or an antigen, and the marker reagent is an antibody reagent provided with a luminescent marker or an antigen reagent provided with a luminescent marker. The first antigen-antibody binder contained in the first reaction solution is generated by an antigen-antibody binding reaction among the sample, the magnetic bead reagent and the marker reagent, and the optical signal detected by the sample detection device 300 is an optical signal generated by the luminescent substrate reagent in the first antigen-antibody binder under catalysis of the luminescent marker.

Or, as an alternative implementation, the reagent dispensing device 200 is further configured to dispense a magnetic bead reagent and a marker reagent to the reaction vessel 20 before the magnetic separation cleaning operation. The magnetic bead reagent is provided with an antibody or an antigen, the marker reagent is an antibody reagent provided with a luminescent marker or an antigen reagent provided with a luminescent marker. The first antigen-antibody binder contained in the first reaction solution is generated by an antigen-antibody binding reaction between the sample and the magnetic bead reagent. The first reaction solution further contains a second antigen-antibody binder generated by an antigen-antibody binding reaction between the marker reagent and the magnetic bead reagent, and the optical signal detected by the sample detection device 300 is an optical signal generated by the luminescent substrate reagent in the second antigen-antibody binder under catalysis of the luminescent marker.

Besides the above descriptions, other parts of the sample analyzer 10 according to this embodiment may refer to the first embodiment, which are not described in detail here.

### Fourth embodiment:

The sample analyzer 10 according to this embodiment differs from the first embodiment mainly in that the cleaning liquid dispensing device 400 in this embodiment is not necessarily provided with both heating and cooling functions, and instead, it only requires reducing the liquid path of the non-backflow section.

Specifically, the sample analyzer 10 according to this embodiment includes a sample dispensing device 100, a reagent dispensing device 200, a sample detection device 300, a magnetic separation device 500, a cleaning liquid dispensing device 400, a substrate dispensing device 101 and a controller 102. The sample dispensing device 100 is configured to aspirate a sample from a sample vessel and dispense at least a part of the aspirated sample to a reaction vessel 20. The reagent dispensing device 200 is configured to aspirate a reagent from a reagent vessel and dispense the aspirated reagent to the reaction vessel 20. The reaction vessel 20 is at least configured to provide a reaction place for the sample and the reagent, so that a first reaction solution containing at least a first antigen-antibody binder is generated by an antigen-antibody binding reaction between a target antigen in the sample and an antibody in the reagent or between a target antibody in the sample and an antigen in the reagent. The cleaning liquid dispensing device 400 is configured to provide a cleaning liquid within a target temperature range. The magnetic separation device 500 is configured to perform a magnetic separation cleaning operation on the first reaction solution through the cleaning liquid provided by the cleaning liquid dispensing device 400 that is within the target temperature range, to prepare a second reaction solution. The substrate dispensing device 101 is configured to dispense a luminescent substrate reagent to the second reaction solution prepared by the magnetic separation cleaning operation, to prepare a third reaction solution. the sample detection device 300 is configured to perform an optical signal detection on the third reaction solution. The controller 102 is configured to control operations of the sample dispensing device 100, the reagent dispensing device 200, the sample detection device 300, the magnetic separation device 500, the substrate dispensing device 101 and the cleaning liquid dispensing device 400, and configured to analyze information of the optical signal detected by the sample detection device 300, so as to obtain a content of the target antigen in the sample or a content of the target antibody in the sample. The cleaning liquid dispensing device 400 includes a heat exchanger 410, a liquid path device 420 and a power device 430. The liquid path device 420 is configured to provide a flow path for a cleaning liquid, and at least a part of the liquid path device 420 is in contact with the heat exchanger 410, so as to exchange heat with the cleaning liquid in the liquid path device 420. The power device 430 is connected to the liquid path device 420 to drive, under control of the controller 102, the liquid path device 420 to aspirate a cleaning liquid from a cleaning liquid container 30, and drive, under control of the controller 102, the liquid path device 420 to dispense the cleaning liquid that reaches a temperature within the target temperature range through heat exchange with the heat exchanger 410, to the first reaction solution in the magnetic separation device 500. The liquid path device 420 includes a main branch 422, a backflow branch 425, a liquid injection branch 423, a switching device 424 and a confluence component 421. One end of the main branch 422 is configured to aspirate the cleaning liquid from the cleaning liquid container 30, the other end of the main branch 422 is connected to the switching device 424 that switches between the backflow branch 425 and the liquid injection branch 423, and at least a part of the main branch 422 forms a heat exchange tube section 4201 in contact with the heat exchanger 410, so as to allow the cleaning liquid in the heat exchange tube section 4201 to exchange heat with the heat exchanger 410. The power device 430 is connected to the main branch 422. The liquid injection branch 423 is configured to dispense the cleaning liquid. The switching device 424 is formed with a liquid inlet *a* and a liquid outlet *b.* The liquid inlet *a* is configured to enable the cleaning liquid after heat exchange through the heat exchanger 410 to flow into the switching device 424. A backflow port *c* is configured to enable the cleaning liquid flowing into the switching device 424 to flow back to the main branch 422 through the backflow branch 425, so as to exchange heat with the heat exchanger 410. The liquid outlet *b* is configured to output the cleaning liquid to the liquid injection branch 423. The switching device 424 is mounted on the confluence component 421. The liquid outlet *b* is abutted against the confluence component 421. The confluence component 421 mainly plays a function of confluence, and a channel enabling the cleaning liquid to flow is formed inside the confluence component 421. The confluence component 421 functions to deliver the cleaning liquid through a rigid structure instead of a pipeline, has a compact structure and is convenient for installation. In this implementation, the switching device 424 is directly mounted on the confluence component 421, so that on one hand, a mounting bracket of the switching device 424 may be omitted; on the other hand, a pipeline between the switching device 424 and the confluence component 421 may be omitted, further simplifying structure of the sample analyzer 10. Furthermore, it is beneficial to reduce an amount of non-backflow liquid, and further ensure accuracy and reliability of the temperature of the cleaning liquid supplied for each detection. In a specific application, a liquid path assembly may deliver the cleaning liquid to the heat exchanger 410 to exchange heat under driving of the power device 430, so that the cleaning liquid reaches a temperature within the target temperature range, and then dispense the cleaning liquid that is within the target temperature range to the first reaction solution in the magnetic separation device 500.

As an implementation, the switching device 424 is a multi-way valve, and the multi-way valve is provided with at least two operation positions and at least three interfaces. The liquid inlet *a*, the backflow port c and the liquid outlet *b* are three interfaces of the multi-way valve respectively.

As an implementation, the switching device 424 is a two-position three-way valve, and the liquid inlet *a*, the backflow port *c* and the liquid outlet *b* are three interfaces of the two-position three-way valve respectively.

As an implementation, the backflow port *c* is also abutted against the confluence component 421. The backflow branch 425 includes a backflow guide channel 4252 formed in the confluence component 421 and a backflow pipeline 4251 at least partially arranged outside the confluence component 421. Each of two ends of the backflow guide channel 4252 is communicated with a respective one of an input end of the backflow pipeline 4251 and the backflow port *c,* so that the cleaning liquid flowing out of the backflow port *c* flows to the backflow pipeline 4251 through the backflow guide channel 4252, and an output end of the backflow pipeline 4251 is communicated with the cleaning liquid container 30.

As an implementation, the liquid inlet *a* is also abutted against the confluence component 421. The main branch 422 includes a liquid inlet guide channel 4222 formed in the confluence component 421 and a main liquid delivery pipeline 4221 arranged outside the confluence component 421. The main liquid delivery pipeline 4221 extends from the cleaning liquid container 30 to the liquid inlet guide channel 4222. At least a part of the main liquid delivery pipeline 4221 forms the heat exchange tube section 4201, each of two ends of the liquid inlet guide channel 4222 is communicated with a respective one of an output end of the main liquid delivery pipeline 4221 and the liquid inlet *a*, so that the cleaning liquid flowing out of the main liquid delivery pipeline 4221 flows to the switching device 424 through the liquid inlet guide channel 4222.

As an implementation, the liquid injection branch 423 includes a liquid injection guide channel 4232 formed in the confluence component 421 and a first liquid injection component 4231 at least partially arranged outside the confluence component 421 to dispense the cleaning liquid, each of two ends of the liquid injection guide channel 4232 is communicated with a respective one of the liquid outlet *b* and the first liquid injection component 4231, that is, the liquid injection guide channel 4232 is connected between the liquid outlet *b* and the first liquid injection component 4231.

As an implementation, the first liquid injection component 4231 penetrates into the confluence component 421 and is fixed to the confluence component 421.

As an implementation, a liquid path formed by the liquid injection branch 423 has a length less than or equal to 200 mm.

As an implementation, the liquid path formed by the liquid injection branch 423 has a length less than or equal to 20 mm.

As an implementation, a volume of the liquid injection branch 423 is less than or equal to 10% of a liquid amount of the cleaning liquid that is dispensed by the liquid injection branch 423 into the first reaction solution in the magnetic separation device 500 at one time.

As an implementation, a volume of the liquid injection branch 423 is less than or equal to 5% of the liquid amount of the cleaning liquid that is dispensed by the liquid injection branch 423 into the first reaction solution in the magnetic separation device 500 at one time.

As an implementation, the magnetic separation device 500 includes a magnetic separation disk 510 and a lid 520. The magnetic separation disk 510 is configured to accommodate multiple reaction vessels 520. The lid 520 covers the magnetic separation disk 510. The liquid path device 420 further includes a main branch 422, a confluence component 421 and a liquid injection branch 423. The liquid injection branch 423 includes a liquid injection guide channel 4232 formed in the confluence component 421 and a first liquid injection component 4231 at least partially arranged outside the confluence component 421 to dispense the cleaning liquid. The main branch 422 is communicated with the first liquid injection component 4231 through the confluence component 421. The confluence component 421 is mounted on the lid 520.

As an implementation, the reagent dispensing device 200 is further configured to dispense a magnetic bead reagent and a marker reagent to the reaction vessel 20 before the magnetic separation cleaning operation. The magnetic bead reagent is provided with an antibody or an antigen, and the marker reagent is an antibody reagent provided with a luminescent marker or an antigen reagent provided with a luminescent marker. The first antigen-antibody binder contained in the first reaction solution is generated by an antigen-antibody binding reaction among the sample, the magnetic bead reagent and the marker reagent, and the optical signal detected by the sample detection device 300 is an optical signal generated by the luminescent substrate reagent in the first antigen-antibody binder under catalysis of the luminescent marker.

Or, as an alternative implementation, the reagent dispensing device 200 is further configured to dispense a magnetic bead reagent and a marker reagent to the reaction vessel 20 before the magnetic separation cleaning operation. The magnetic bead reagent is provided with an antibody or an antigen, and the marker reagent is an antibody reagent provided with a luminescent marker or an antigen reagent provided with a luminescent marker. The first antigen-antibody binder contained in the first reaction solution is generated by an antigen-antibody binding reaction between the sample and the magnetic bead reagent, the first reaction solution further contains a second antigen-antibody binder generated by an antigen-antibody binding reaction between the marker reagent and the magnetic bead reagent, and the optical signal detected by the sample detection device 300 is an optical signal generated by the luminescent substrate reagent in the second antigen-antibody binder under catalysis of the luminescent marker.

As an implementation, the controller 102 is further configured to control the target temperature range for the magnetic separation cleaning operation for the at least one sample detection item to be within 10°C to 35°C, that is, the temperature in the target temperature range for each of the at least one sample detection item is equal to or greater than 10°C and less than or equal to 35°C.

As an implementation, the controller 102 is further configured to control the target temperature range for the magnetic separation cleaning operation for the at least one sample detection item to be within 15°C to 30°C, that is, the temperature in the target temperature range for each of the at least one sample detection item is equal to or greater than 15°C and less than or equal to 30°C.

As an implementation, the controller 102 is further configured to control the target temperature range for the magnetic separation cleaning operation for the at least one sample detection item to be within 24°C to 30°C, that is, the temperature in the target temperature range for each of the at least one sample detection item is equal to or greater than 24°C and less than or equal to 30°C.

Besides the above descriptions, other parts of the sample analyzer 10 according to this embodiment may refer to the first embodiment, which are not described in detail here.

The above descriptions are only preferred embodiments of the present invention, and are not intended to limit the patent scope of the present invention. Any equivalent structural transformation made by using contents of the description and drawings of the present invention under the inventive concept of the present invention, or direct/indirect present invention of the present invention in other related technical fields, is included in the patent scope of protection of the present invention.

## Claims

1. A sample analyzer (10), **characterized in that** the sample analyzer (10) comprises a sample dispensing device (100), a reagent dispensing device (200), a sample detection device (300), a magnetic separation device (500), a substrate dispensing device (101), a cleaning liquid dispensing device (400) and a controller (102), wherein
the sample dispensing device (100) is configured to aspirate a sample from a sample vessel and dispense at least a part of the aspirated sample to a reaction vessel (20);
the reagent dispensing device (200) is configured to aspirate a reagent from a reagent vessel and dispense at least a part of the aspirated reagent to the reaction vessel (20), the reaction vessel (20) is at least configured to provide a reaction place for the sample and the reagent, so that a first reaction solution containing at least a first antigen-antibody binder is generated by an antigen-antibody binding reaction between a target antigen in the sample and an antibody in the reagent or between a target antibody in the sample and an antigen in the reagent;
the cleaning liquid dispensing device (400) comprises a heat exchanger (410), a liquid path device (420) and a power device (430), the heat exchanger (410) is configured to perform heating and cooling respectively under control of the controller (102), the liquid path device (420) is configured to provide a flow path for a cleaning liquid, and at least a part of the liquid path device (420) is in contact with the heat exchanger (410), so as to allow the cleaning liquid in the liquid path device (420) to be heated or cooled by the heat exchanger (410) to reach a temperature within a target temperature range, the power device (430) is connected to the liquid path device (420), to drive, under control of the controller (102), the liquid path device (420) to aspirate the cleaning liquid from a cleaning liquid container (30), and drive, under control of the controller (102), the liquid path device (420) to dispense the cleaning liquid that reaches a temperature within the target temperature range through heating or cooling by the heat exchanger (410), to the first reaction solution in the magnetic separation device (500);
the magnetic separation device (500) is configured to perform a magnetic separation cleaning operation on the first reaction solution through the cleaning liquid provided by the cleaning liquid dispensing device (400) that is within the target temperature range, so as to prepare a second reaction solution;
the substrate dispensing device (101) is configured to dispense a luminescent substrate reagent to the second reaction solution prepared by the magnetic separation cleaning operation, so as to prepare a third reaction solution;
the sample detection device (300) is configured to perform an optical signal detection on the third reaction solution;
the controller (102) is configured to control operations of the sample dispensing device (100), the reagent dispensing device (200), the sample detection device (300), the magnetic separation device (500), the substrate dispensing device (101) and the cleaning liquid dispensing device (400), and configured to analyze information of an optical signal detected by the sample detection device (300), so as to obtain a content of the target antigen in the sample or a content of the target antibody in the sample.

2. The sample analyzer (10) of claim 1, wherein the reagent dispensing device (200) is further configured to dispense a magnetic bead reagent and a marker reagent to the reaction vessel (20) before the magnetic separation cleaning operation, the magnetic bead reagent is provided with the antibody or the antigen, the marker reagent is an antibody reagent provided with a luminescent marker or an antigen reagent provided with a luminescent marker; the first antigen-antibody binder contained in the first reaction solution is generated by an antigen-antibody binding reaction among the sample, the magnetic bead reagent and the marker reagent, and the optical signal detected by the sample detection device (300) is an optical signal generated by the luminescent substrate reagent in the first antigen-antibody binder under catalysis of the luminescent marker; or
the reagent dispensing device (200) is further configured to dispense a magnetic bead reagent and a marker reagent to the reaction vessel (20) before the magnetic separation cleaning operation, the magnetic bead reagent is provided with the antibody or the antigen, the marker reagent is an antibody reagent provided with a luminescent marker or an antigen reagent provided with a luminescent marker; the first antigen-antibody binder contained in the first reaction solution is generated by an antigen-antibody binding reaction between the sample and the magnetic bead reagent, the first reaction solution further contains a second antigen-antibody binder generated by an antigen-antibody binding reaction between the marker reagent and the magnetic bead reagent, and the optical signal detected by the sample detection device (300) is an optical signal generated by the luminescent substrate reagent in the second antigen-antibody binder under catalysis of the luminescent marker.

3. The sample analyzer (10) of claim 1, wherein the controller (102) is further configured to control a target temperature range for the magnetic separation cleaning operation for at least one sample detection item to be within 10°C to 35°C, and preferably within 15°C to 30°C.

4. The sample analyzer (10) of claim 1, wherein the heat exchanger (410) comprises a housing (415) and a heat exchange component (412) arranged in the housing (415), the liquid path device (420) comprises a heat exchange tube section (4201), and the liquid path device (420) is at least partially arranged in the housing (415) and in contact with the heat exchange component (412), or
wherein the heat exchanger (410) comprises a heat exchange component (412) and a single energy generation component (411), the single energy generation component (411) is configured to be switched between cooling and heating under control of the controller (102), the heat exchange component (412) is heat-conductively connected to the single energy generation component (411), the liquid path device (420) comprises a heat exchange tube section (4201), the heat exchange tube section (4201) is in contact with the heat exchange component (412), so as to enable the cleaning liquid in the heat exchange tube section (4201) to exchange heat with the heat exchange component (412).

5. The sample analyzer (10) of claim 4, wherein the single energy generation component (411) is a semiconductor cooling fin (4111), the semiconductor cooling fin (4111) is configured to be switched between cooling and heating under control of the controller (102), the semiconductor cooling fin (4111) is heat-conductively connected to the heat exchange component (412);
wherein a groove (4121) is formed at a side of the heat exchange component (412), and
wherein the heat exchange tube section (4201) is at least partially embedded in the groove (4121) and in contact with an inner wall of the groove (4121), and/or the heat exchange tube section (4201) is spirally wound on the heat exchange component (412).

6. The sample analyzer (10) of claim 5, wherein the cleaning liquid dispensing device (400) further comprises a temperature detection component (440), the temperature detection component (440) is configured to detect a temperature of the heat exchange component (412) or detect a temperature of the cleaning liquid outputted through the heat exchange tube section (4201);
the controller (102) is further configured to control operation of the semiconductor cooling fin (4111) according to temperature information fed back by the temperature detection component (440),
preferably, controlling the operation of the semiconductor cooling fin (4111) according to the temperature information fed back by the temperature detection component (440) comprises:
while performing a magnetic separation cleaning operation for a sample detection item, controlling the semiconductor cooling fin (4111) to perform cooling when the temperature fed back by the temperature detection component (440) is higher than an upper limit value of a target temperature range for the magnetic separation cleaning operation for the sample detection item, so that the heat exchange component (412) cools the cleaning liquid flowing through the heat exchange tube section (4201); and controlling the semiconductor cooling fin (4111) to perform heating when the temperature fed back by the temperature detection component (440) is lower than a lower limit value of the target temperature range for the magnetic separation cleaning operation for the sample detection item, so that the heat exchange component (412) heats the cleaning liquid flowing through the heat exchange tube section (4201).

7. The sample analyzer (10) of any one of claims 1 to 4, wherein the controller (102) is further configured to control the magnetic separation device (500) to perform a magnetic separation cleaning operation for each of one or more sample detection items of a first type and then control the magnetic separation device (500) to perform a magnetic separation cleaning operation for each of one or more sample detection items of a second type, when a plurality of sample detection items for a same batch of samples are to be performed,
wherein the one or more sample detection items of the first type and the one or more sample detection items of the second type meet at least one of following conditions: a temperature control requirement of the one or more sample detection items of the first type is lower than that of the one or more sample detection items of the second type; or a difference between a target temperature range required to perform the magnetic separation cleaning operation for each of the one or more sample detection items of the first type and ambient temperature is smaller than a difference between a target temperature range required to perform the magnetic separation cleaning operation for each of the one or more sample detection items of the second type and ambient temperature.

8. The sample analyzer (10) of claim 1, wherein the liquid path device (420) comprises a main branch (422), a backflow branch (425), a liquid injection branch (423) and a switching device (424), one end of the main branch (422) is configured to aspirate the cleaning liquid from the cleaning liquid container (30), the other end of the main branch (422) is connected to the switching device (424) that switches between the backflow branch (425) and the liquid injection branch (423), and at least a part of the main branch (422) forms a heat exchange tube section (4201) that is in contact with the heat exchanger (410), so as to allow the cleaning liquid in the heat exchange tube section (4201) to be heated or cooled by the heat exchanger (410);
the power device (430) is connected to the main branch (422);
the liquid injection branch (423) is configured to dispense the cleaning liquid;
the backflow branch (425) forms a circulation loop with the main branch (422) and the cleaning liquid container (30), or the backflow branch (425) forms a circulation loop with the main branch (422), so that the cleaning liquid outputted from the main branch (422) is guided by the backflow branch (425) to circulate through the heat exchanger (410), so as to exchange heat.

9. The sample analyzer (10) of claim 8, wherein a liquid path formed by the liquid injection branch (423) has a length less than or equal to 200 mm, and preferably, less than or equal to 20 mm.

10. The sample analyzer (10) of claim 8, wherein a volume of the liquid injection branch (423) is less than or equal to 10%, and preferably, 5% of a liquid amount of the cleaning liquid that is dispensed by the liquid injection branch (423) into the first reaction solution in the magnetic separation device (500) at one time.

11. The sample analyzer (10) of claim 8, wherein the switching device (424) is formed with a liquid inlet (*a*), a backflow port (*c*) and a liquid outlet (*b*), the liquid inlet (*a*) is configured to enable the cleaning liquid after heat exchange through the heat exchanger (410) to flow into the switching device (424), the backflow port (*c*) is configured to enable the cleaning liquid flowing into the switching device (424) to flow back to the main branch (422) through the backflow branch (425), so as to exchange heat with the heat exchanger (410), and the liquid outlet (b) is configured to output the cleaning liquid to the liquid injection branch (423);
the liquid path device (420) further comprises a confluence component (421), the switching device (424) is mounted on the confluence component (421), the liquid outlet (b) is abutted against the confluence component (421), the liquid injection branch (423) comprises a liquid injection guide channel (4232) formed in the confluence component (421) and a first liquid injection component (4231) at least partially arranged outside the confluence component (421) to dispense the cleaning liquid, and each of two ends of the liquid injection guide channel (4232) is communicated with a respective one of the liquid outlet (b) and the first liquid injection component (4231).

12. The sample analyzer (10) of claim 11, wherein the switching device (424) is a multi-way valve, the backflow port (c) is also abutted against the confluence component (421), the backflow branch (425) comprises a backflow guide channel formed in the confluence component (421) and a backflow pipeline at least partially arranged outside the confluence component (421), each of two ends of the backflow guide channel is communicated with a respective one of an input end of the backflow pipeline and the backflow port (c), so that the cleaning liquid flowing out of the backflow port (c) flows to the backflow pipeline through the backflow guide channel, and an output end of the backflow pipeline is communicated with the cleaning liquid container (30) or the main branch (422); and/or
the switching device (424) is a multi-way valve, the liquid inlet is also abutted against the confluence component (421), the main branch (422) comprises a liquid inlet guide channel (4222) formed in the confluence component (421) and a main liquid delivery pipeline (4221) arranged outside the confluence component (421), the main liquid delivery pipeline (4221) extends from the cleaning liquid container (30) to the liquid inlet guide channel (4222), at least a part of the main liquid delivery pipeline (4221) forms the heat exchange tube section (4201), each of two ends of the liquid inlet guide channel (4222) is communicated with a respective one of an output end of the main liquid delivery pipeline (4221) and the liquid inlet (*a*), so that the cleaning liquid flowing out of the main liquid delivery pipeline (4221) flows to the switching device (424) through the liquid inlet guide channel (4222).

13. The sample analyzer (10) of claim 11, wherein an orthographic projection of the liquid outlet (b) on the confluence component (421) does not overlap with an orthographic projection of the first liquid injection component (4231) on the confluence component (421); the liquid injection guide channel (4232) comprises a first channel part and a second channel part, one end of the first channel part is communicated with the liquid outlet (b), the second channel part extends from the other end of the first channel part to the first liquid injection component (4231), and wherein an angle greater than 0° and less than 180° is formed at a junction of the first channel part and the second channel part; and/or the first liquid injection component (4231) is arranged obliquely relative to a vertical direction, and
wherein the first liquid injection component (4231) penetrates into the confluence component (421).

14. The sample analyzer (10) of any one of claims 1 to 4 or any one of claims 8 to 13, wherein the controller (102) is further configured to configure different temperature control parameters for at least two sample detection items according to different sample detection items and/or different liquid injection amounts of the cleaning liquid, and the different temperature control parameters comprise at least one of following different parameters: operation power of the heat exchanger (410), a liquid amount of the cleaning liquid flowing back through a backflow branch (425) before dispensing the cleaning liquid to the first reaction solution while performing a single sample detection item, or a liquid amount of the cleaning liquid flowing through the heat exchanger (410) before dispensing the cleaning liquid to the first reaction solution while performing the single sample detection item; or
wherein the controller (102) is further configured to configure different temperature control parameters for at least two sample detection items according to different ambient temperatures, and the different temperature control parameters comprise at least one of following different parameters: operation power of the heat exchanger (410), a liquid amount of the cleaning liquid flowing back through a backflow branch (425) before dispensing the cleaning liquid to the first reaction solution of a first sample detection item while performing a plurality of sample detection items for a same batch of samples, a liquid amount of the cleaning liquid flowing through the heat exchanger (410) before dispensing the cleaning liquid to the first reaction solution of the first sample detection item while performing a plurality of sample detection items for a same batch of samples, a liquid amount of the cleaning liquid flowing through the backflow branch (425) while performing a plurality of sample detection items for a same batch of samples, or a frequency of the cleaning liquid flowing through the backflow branch (425) while performing a plurality of sample detection items for a same batch of samples.

15. The sample analyzer (10) of any one of claims 1 to 4 or any one of claims 8 to 13, wherein the magnetic separation device (500) comprises a holding assembly, a transfer assembly, at least one liquid aspiration assembly (530), at least one first magnetic assembly (540) and at least one second magnetic assembly (550), the first magnetic assembly (540) includes a pair of first magnetic devices (541) arranged at an interval, a first magnetic attraction position (501) is formed between the pair of first magnetic devices (541) arranged at the interval or at a same side of the pair of first magnetic devices (541) arranged at the interval, and the first magnetic assembly (540) is configured to perform a first magnetic attraction action on the first reaction solution in a reaction vessel (20) located at the first magnetic attraction position (501), so as to form two spaced magnetic bead groups (40) on an inner side wall of the reaction vessel (20) located at the first magnetic attraction position (501) by attraction;
the second magnetic assembly (550) is a single second magnetic device (551), a first liquid aspiration position (502) is formed at a side of the single second magnetic device (551), the second magnetic assembly (550) is configured to perform a second magnetic attraction action on the first reaction solution in a reaction vessel (20) located at the first liquid aspiration position (502), so as to form a single magnetic bead group (40) on an inner side wall of the reaction vessel (20) located at the first liquid aspiration position (502) by attraction;
the liquid aspiration assembly (530) is configured to perform a first liquid aspiration action on the first reaction solution in the reaction vessel (20) located at the first liquid aspiration position (502);
the holding assembly is at least configured to hold the reaction vessel (20) located at the first magnetic attraction position (501) and hold the reaction vessel (20) located at the first liquid aspiration position (502);
the transfer assembly is at least configured to transfer a reaction vessel (20) loaded with the first reaction solution to the first magnetic attraction position (501), and transfer a reaction vessel (20) after completing the first magnetic attraction action to the first liquid aspiration position (502).
